(19) Europäisches Patentamt / European Patent Office / Office européen des brevets

(11) **EP 2 400 882 B1**

(12) **EUROPEAN PATENT SPECIFICATION**

(45) Date of publication and mention
of the grant of the patent:
**31.05.2017  Bulletin 2017/22**

(21) Application number: **10745888.7**

(22) Date of filing: **25.02.2010**

(51) Int Cl.:
***G06F 19/00*** *(2011.01)*      ***A61M 5/00*** *(2006.01)*
***A61B 5/00*** *(2006.01)*

(86) International application number:
**PCT/IL2010/000161**

(87) International publication number:
**WO 2010/097796 (02.09.2010 Gazette 2010/35)**

(54) **METHOD AND SYSTEM FOR AUTOMATIC MONITORING OF DIABETES RELATED TREATMENTS**

VERFAHREN UND SYSTEM ZUR AUTOMATISCHEN ÜBERWACHUNG VON
DIABETESBEDINGTEN BEHANDLUNGEN

PROCÉDÉ ET SYSTÈME DE SURVEILLANCE AUTOMATIQUE DE TRAITEMENTS LIÉS AU
DIABÈTE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **26.02.2009  US 155556 P
30.09.2009  US 247017 P
03.02.2010  US 300874 P**

(43) Date of publication of application:
**04.01.2012  Bulletin 2012/01**

(73) Proprietor: **DreaMed Diabetes Ltd
6971054 Tel Aviv (IL)**

(72) Inventors:
 • **ATLAS, Eran
   Petach Tiqwa 49720 (IL)**
 • **NIMRI, Revital
   49550 Petach Tiqwa (IL)**
 • **MILLER, Shahar
   49728 Petah Tikva (IL)**
 • **GRUNBERG, Eli
   Patech Tiqwa 49065 (IL)**
 • **PHILLIP, Moshe
   53631 Givaataim (IL)**

(74) Representative: **Grünecker Patent- und
Rechtsanwälte
PartG mbB
Leopoldstraße 4
80802 München (DE)**

(56) References cited:
**WO-A2-2007/149533      US-A1- 2006 173 406
US-A1- 2007 066 873     US-A1- 2008 206 799
US-A1- 2008 294 294     US-B1- 6 572 542
US-B2- 6 575 905**

 • **SH YASINI ET AL: "Active insulin infusion using
   fuzzy-based closed-loop control", INTELLIGENT
   SYSTEM AND KNOWLEDGE ENGINEERING,
   2008. ISKE 2008. 3RD INTERNATIONAL
   CONFERENCE ON, IEEE, PISCATAWAY, NJ,
   USA, 17 November 2008 (2008-11-17), pages
   429-434, XP031381974, ISBN: 978-1-4244-2196-1**
 • **ROB SUSANTO-LEE ET AL: "Simulation of
   fuzzy-modified expert PID algorithms for blood
   glucose control", CONTROL, AUTOMATION,
   ROBOTICS AND VISION, 2008. ICARCV 2008.
   10TH INTERNATIONAL CONFERENCE ON, IEEE,
   PISCATAWAY, NJ, USA, 17 December 2008
   (2008-12-17), pages 1583-1589, XP031433829,
   ISBN: 978-1-4244-2286-9**
 • **Rodrigo E Teixeira ET AL: "The Next Generation
   of Artificial Pancreas Control Algorithms Journal
   of Diabetes Science and Technology", J Diabetes
   Sci Technol, 1 January 2008 (2008-01-01), pages
   105-112, XP055214783, Retrieved from the
   Internet:
   URL:http://www.ncbi.nlm.nih.gov/pmc/articl
   es/PMC2769707/pdf/dst-02-0105.pdf [retrieved
   on 2015-09-21]**

**Description**

**FIELD OF THE INVENTION**

This invention is in the field of monitoring diabetes-related treatment, and relates to a method and system for automatic monitoring of diabetes related treatments.

**REFERENCES**

The following references are considered to be pertinent for the purpose of understanding the background of the present invention:

1. Steil G, Panteleon A, Rebrin K. Closed-loop insulin delivery-the path to physiological glucose control. Adv Drug Deliv Rev 2004; 56:125-144
2. Parker R, Doyle Fr, Peppas N. A model-based algorithm for blood glucose control in type I diabetic patients. IEEE Trans Biomed Eng 1999; 46:148-157
3. Hovorka R, Chassin L, Wilinska M, Canonico V, Akwi J, Federici M, Massi-Benedetti M, Hutzli I, Zaugg C, Kaufmann H, Both M, Vering T, Schaller H, Schaupp L, Bodenlenz M, Pieber T. Closing the loop: the adicol experience. Diabetes Technol Ther 2004; 6:307-318
4. Hovorka R, Canonico V, Chassin L, Haueter U, Massi-Benedetti M, Orsini Federici M, Pieber T, Schaller H, Schaupp L, Vering T, Wilinska M. Nonlinear model predictive control of glucose concentration in subjects with type 1 diabetes. Physiol Meas 2004; 25:905-920
5. Magni L, Raimondo D, Bossi L, Dalla Man C, De Nicolao G, Kovatchev B, Cobelli C. Model Predictive Control of Type 1 Diabetes: An In Silico Trai. J Diabetes Sci Technol 2007; 1:804-812
6. Pedrycz W, Gomide F. Fuzzy Systems Engineering Towards Human-Centeric Computing. Hoboken, New Jersy, John Wiley & Sons, Inc., 2007
7. Sincanandam SN, Sumathi S, Deepa SN. Introduction to Fuzzy Logic using MATLAB. Verlag Berlin Heidelberg, Springer, 2007
8. Sparacino G, Zanderigo F, Corazza S, Maran A, Facchinetti A, Cobelli C. Glucose concentration can be predicted ahead in time from continuous glucose monitoring sensor time-series. IEEE Trans Biomed Eng 2007; 54:931-937
9. Magni L, Raimondo D, Dalla Man C, Breton M, Patek S, De Nicolao G, Cobelli C, Kovatchev B. Evaluating the Efficacy of Closed-Loop Glucose Regulation via Control-Variability Grid Analysis. J Diabetes Sci Technol 2008; 2:630-635
10. Standards of medical care in diabetes--2009. Diabetes Care 2009; 32 Suppl 1:S13-61

**BACKGROUND OF THE INVENTION**

Diabetes mellitus, usually called diabetes, is a disease in which an individual's pancreas does not make enough insulin or the individual's body cannot use normal amounts of insulin properly. Insulin, a hormone produced by the pancreas, helps maintain normal blood sugar levels.

Type 1 diabetes is a chronic, life-threatening disease that is caused by failure of the pancreas to deliver the hormone insulin, which is otherwise made and secreted by the beta cells of the pancreatic islets of Langerhans. With the resulting absence of endogenous insulin, people with type 1 diabetes cannot regulate their blood glucose to euglycemic range without exogenous insulin administration. However, it is critical to provide accurate insulin dosing, so as to minimize and whenever possible eliminate low or high blood glucose levels. Both high glucose levels, known as hyperglycemia, and low glucose levels, known as hypoglycemia, can have debilitating and deleterious consequences. Hypoglycemia may result in a coma and can cause acute complications, including brain damage and paralysis. While severe hyperglycemia can also result in a coma, mild chronic hyperglycemia potentially results in long-term, deleterious, and even life-threatening complications, such as vascular disease, renal complications, vision problems, nerve degeneration, and skin disorders.

Therefore, it is necessary for people with type 1 diabetes to monitor their blood glucose and administer exogenous insulin several times a day in a relentless effort to maintain their blood glucose near euglycemic range. This is a demanding, painstaking regimen. Even those who successfully adhere to the regimen are burdened by it to varying degrees and often still struggle with maintaining good glycemic control. Those who do not follow a regimen are at risk for severe complications.

Type 1 patients usually use two delivery regimes to deliver insulin. These régimes came from the physiological method the pancreas deliver insulin: (1) a constant basal rate for maintaining a constant blood glucose level- a small amount of insulin is continuously delivered to the blood stream in order to maintain normal glucose levels. This level could be high, low or in normal range; (2) A bolus for compensating for consuming a meal or to correct high blood glucose level - quick delivery of large amount of insulin (usually this amount is delivered in a matter of minutes).

The core of the ideal Artificial Pancreas (AP) system is the control algorithm which automatically modulates insulin delivery (optionally other hormones) according to measured glucose levels. Current state of the art control algorithms for clinical use are focused on either traditional control theory or relayed on set of equations which describes the glucose-insulin dynamics.

The artificial pancreas systems are usually based either on traditional linear control theory or rely on mathematical models of glucose-insulin dynamics. The most common techniques are based one proportional-integral-derivative control (PID) [1] and model predictive control (MPC) [2-5]. However, the nonlinearity, complexity and uncertainty of the biological system along with the inherited delay and deviation of the measuring devices, makes difficult to define a model and correctly evaluate the physiological behavior of the individual patient [1-3, 5]. In addition, because most of the control algorithms are not amenable to multiple inputs and multiple outputs, the measured blood glucose level is generally, the only input implemented, and insulin delivery is the only implemented output.

The PID control algorithm produces an insulin profile similar to the secretion profile done by the beta cells extrapolated by three components [1]. Some controllers include a subset of components, for example, a proportional-derivative (PD) controller includes the proportional and derivative components to improve robustness. Both PID and PD use the measured BG level as the only input and ignore other parameters, such as previous administered insulin doses. The MPC is based on mathematical model and equations which describes the glucose level response to different insulin doses and carbohydrate consumption. As the response to different insulin treatment is implied by the set of equations, an optimal treatment may be found and applied accordingly. The mathematical model is subject specific, and depends upon system identification phase to estimate the required parameters [3]. The main drawback of MPC in relation to glucose control is the need of a good crisp mathematical model and a good method to estimate its parameters in order to describe the physiological behavior of the patient. However, due to the complexity of biological systems, these models are subject to extreme uncertainties, which make it very hard to evaluate and define the model properly. Most of the attempts in the past to develop Subcutaneous (S.C.) closed loop system used linear control methodology to control the non-linear biological system [2, 5] and disregarded the uncertainty of the biological system and the measuring devices. In addition, it is quite difficult to implement multiple inputs and multiple outputs using these methods.

A system and method for controlling the dispensing of insulin is known from WO 2007/149533.

## GENERAL DESCRIPTION OF THE INVENTION

The invention provides a system and a method for automatic monitoring of diabetes-related treatment of a patient according to claims 1 and 11, respectively.

The current diabetes treatment technologies, such as subcutaneous (S.C) insulin pumps and S.C continuous glucose sensors (CGS), have been shown to be helpful in improving the control of T1DM. Despite this, the potential of these technologies in assisting patients with the day-to-day demands of their diabetes management has not been fulfilled. Therefore, there is a need for an AP system that will mimic the activity of the pancreatic cells and strictly control the patient's BG levels while avoiding severe hypoglycemia events. Such a system may also offer an opportunity to free the patients from the daily burden of dealing with their diabetes.

The present invention provides a closed-loop artificial pancreas system offering the opportunity to mimic the activity of functioning pancreatic beta cells and strictly control the patient's blood glucose levels. The monitoring technique of the present invention analyzes data generated by intravenous and/or subcutaneous drug injection devices and by glucose sensors, and decides the treatment modification by controlling the operation of the drug injection devices.

It should be noted that delay of insulin absorption and the fact that the interstitial fluid does not always correctly represent the blood glucose level, turns the mission of closing the loop into a very challenging one. The ultimate goal in diabetes treatment is the development of an autonomous and automatic monitoring and treatment system that mimics the activity of the pancreatic beta cells. Such system is thus capable of maintaining normal physiologic blood glucose levels and therefore avoids hypoglycemia. The system is fully automated (the patient does not have to give an approval for the dosing suggestions) and analyze glucose dynamics and insulin continuously.

Thus, according to a broad aspect of the invention, there is provided a monitoring system for use in monitoring diabetes treatment of a patient. The system comprises a control unit comprising a first processor module for processing measured data indicative of blood glucose level and generating first processed data indicative thereof, a second processor module comprising at least one fuzzy logic module; the second processor module receives input parameters corresponding to the measured data, the first processed data and a reference data including individualized patient's profile related data, individualized patient's treatment history related data and processes the received data to produce at least one qualitative output parameter indicative of patient's treatment parameters, such that the second processor module determines whether any of the treatment parameters is to be modified.

The monitoring system of the present invention is a computerized system capable of real-time automatic monitoring of a treatment procedure in patients with type 1 diabetes. The monitoring system provides an individualized (subject-specific) control method for automatic glucose regulation in subcutaneous or intravascular sensing and delivery paths. The monitoring technique automatically modulates insulin delivery (and optionally other hormones) according to measured glucose levels and / or other parameters. The system continuously tracks the glucose level and continuously evaluates the active insulin (or other hormones) present in the blood in order to consider additional insulin infusion. By taking the individual subject's treatment history into account, the system of the present invention accurately adjust the control parameters and overcome inter- and intra-patient variability. The monitoring technique of the present invention enables minimizing high glucose peaks while preventing hypoglycemia.

When associated with external glucose sensor and insulin pump, the monitoring system of the present invention is thus operable as a full closed-loop artificial pancreas. The monitoring system comprises *inter alia* functional parts such as a memory utility, and a control unit. The system is used for processing measured data generated by any known suitable measurement device for measuring blood/tissue glucose levels (e.g. by implantable measurement devices) and for controlling any suitable drug injection device (e.g. delivery pump such as subcutaneous insulin pump), therefore a closed-loop analysis of measured data is provided. Therefore, the control unit may be associated with a drug injection device. The control unit is configured and operable to control the operation of the drug injection device. The measured data includes current and past glucose levels relative to a certain time.

Depending on the type of the measurement (continuous or not) and of the injection devices used (implantable or not; operable by signal transmission via wires or wireless), the monitoring system may be equipped with an appropriate data transceiver (communication utility) communicating between the measurement and the injection devices and receiving at least one of the reference data and / or the measured data. The data transceiver is also operable to transmit the at least one output parameter of the control unit to the drug injection device.

It should be understood that in the present invention, the closed-loop analysis is based on a physician approach for decision making with respect to a specific patient under treatment and is adapted to control further treatment accordingly (feedback). This is contrary to the conventional approach used in the systems of the type specified, where mathematical models (such as MPC) are used for evaluating settings of the drug delivery devices from the measured glucose level data.

According to the invention, the second processor module can provide control to range (CRM) output treatment suggestion. The second processor module may include a control to range module (CRM). The *control to range* module or approach provides output treatment suggestion(s) to bring the patient's glucose levels within at least one desired range. The CRM output treatment suggestion comprises at least one of insulin basal rate, insulin bolus or glucagon bolus. The CRM module can be implemented by at least one fuzzy logic module. In some embodiment, the systems and methods of the present invention employs two or more fuzzy logic module (or CRM modules). In some embodiment, a fuzzy logic module (or CRM module) is assigned, configured or adapted to handle an event. According to the invention, the second processor module comprises at least one fuzzy logic module having a modeled structure of rules (or set of rules); the fuzzy logic module utilizes one or more member functions modeled for translating the input parameters into one or more qualitative output parameters. In some embodiments, where two or more fuzzy logic modules are employed, two or more fuzzy logic modules are employed each having either identical and / or different modeled structure of rules, identical and / or different input parameters, identical and / or different qualitative output parameters. The input / output parameter(s) and set of rules can be designed to handle a special event.

The at least one qualitative output parameter of the fuzzy logic module comprises data indicative of at least one treatment parameter of bolus glucagon, bolus insulin and basal insulin.

In some embodiments, the control unit further includes a third processor module receiving the at least one qualitative output parameter of the fuzzy logic module and processing the at least one output parameter to determine whether any of the treatment parameters is to be modified.

The third processor module can include a control to target module (CTM), or "Treatment Jury" that apply further processing and determines the amount of dosing of insulin and/or glucagon to be delivered i.e. determine whether any of the treatment parameters is to be modified. The *control to target* approach enables to bring the patient's glucose level to a specific target level within the desired range or not.

In some embodiments, the CTM applies further processing to the output of the fuzzy logic module (such as that of the CRM module) and determines the amount of dosing of insulin and/or glucagon to be delivered. Therefore, the third processor receives the control to range CRM output treatment suggestion, and determine the amount of dosing in accordance with a glucose target of the patient's profile. The amount may be adjusted in accordance with at least one of patient's insulin or glucagon pharmacodynamics and the measured data.

According to some aspects of the present invention, the system and methods of the present invention are optionally configured and operable to perform a combination of *control to range* and *control to target* approaches to automatically regulate individual glucose levels. The system optionally has individualized prediction tools (of any known type) for predicting the glucose level in blood based on the measured glucose level in tissue and overcome sensing and delivery delays.

The CRM utilizes a fuzzy logic based model ("table of rules") which is configured for receiving quantitative input parameters and qualitative input parameters (measured/calculated) and transform them into qualitative parameters, corresponding to predetermined rules and degree of statistical agreement of the rule. In some embodiments, the predetermined rules are processed in the basis of medical knowledge. Generally, the main elements of a fuzzy logic module are fuzzy sets of multiple inputs and single or multiple outputs, fuzzy rules structured according to the form of IF (input)-THEN (output), and methods of fuzzification and defuzzification to evaluate the fuzzy-rule output based on the input [6,7]. In the present invention, the fuzzy logic module(s) can be used continuously to receive and / or respond to continuously provided input parameters. In some embodiments, the fuzzy logic module(s) of the present invention respond to a special event(s). In this connection, it should be understood that the system of the present invention provides a continuous controller using a fuzzy logic module to determine possible modification of treatment parameters. The fuzzy logic module is not used as a predictor predicting the next blood glucose level based on a mathematical model or as a prediction tool predicting hypoglycemia.

The input parameters received by the second processor module include at least one of the followings: past blood glucose level trend, current blood glucose level (e.g. measured by subcutaneous continuous glucose sensor (CGS)), future blood glucose trend, future blood glucose level.

The quantitative input parameters are preprocessed by the processing module to yield at least one qualitative output parameter that predicts the glucose trace within a predefined prediction horizon. In this connection, it should be understood that, each patient having its own treatment history including several parameters, the prediction of the glucose trace is calculated with respect to the parameters of the treatment history. The fuzzy logic module outputs can be in percents of the individual treatment history.

The CTM aims to bring the patient's glucose to a specific target level. In order to reach the final dosing recommendation, the CTM take into consideration the recommendation of the CRM (in percentage), the predefined glucose target level and an individualized patient's profile related data.

The system uses a processor module to analyze the measured data and utilize such qualitative inputs as the history of treatment for the specific patient (e.g. glucose levels as function of time and insulin treatment history) and the individualized/personalized patient's profile (e.g. sensitivity to insulin/glucagon injection, e.g. tendency to hypoglycemia).

The individualized patient's profile related data comprises parameters selected from at least one of insulin sensitivity, glucagon sensitivity, basal plan, insulin/glucagon pharmacokinetics associated data, glucose target level or target range level, and insulin / glucagon activity model.

The individualized patient's profile related data includes a set of parameters previously calculated or updated/calibrated (learned in real-time) during treatment or during monitoring procedure. In some embodiments, the individualized patient's profile related data can be updated and/or calibrated before and / or during operation.

The calculated parameters may be extracted from pre-recorded data (e.g. from continuous glucose sensor (CGS) readings, glucometer measurements, insulin treatment and activity diary such as meal diary). These parameters are indicative of the patient's condition with respect to a treatment, such as a response time to insulin absorption, insulin sensitivity for meals and glucose levels and preferably also glucagon, all preferably being a function of time and patient's current condition depending on his/her activity. In this connection, it should be noted that the term "insulin/glucagon sensitivity" is referred to the insulin/glucagon correction factor for glucose levels correction for a closed loop session. For an open loop session, the term "insulin sensitivity" includes also the correction of the carbohydrate.

The individualized patient's treatment history related data includes for example the patient's insulin-delivery regimen (insulin basal plan and insulin correction factor and / or insulin carbohydrate ratio) given to the patient at different hours of the day, the insulin pharmacodynamics, and the patient's physical characteristics. The patient's treatment history is updated continuously upon receiving measured data about the patient dynamics during the monitoring/treatment procedure.

Both the patient's treatment history and the performance of control unit are adjustable, enabling the system to deal with inter- and intra-patient variability.

As the invention utilizes the patient's profile, which includes a set of calibratable/updatable parameters, the system applies a self-learning approach for updated the patient's profile based on the executed treatment.

In an example, the system output is aimed at controlling the patient's treatment by injection of both the insulin and glucagon. It should be noted that glucagon can operate as a counter regulatory arm. Glucagon mimics the physiological system in glucose regulation by utilizing the body's own glucose reserves. Furthermore, endogenous glucagon secretion is somewhat compromised in type 1 diabetes. Glucagon thus improves glucose regulation and provides safer operation than could be expected from a closed-loop control system that relies on insulin alone. Moreover, by adding glucagon, the system of the present invention can be more aggressive with insulin dosing resulting in a significantly shorter time to reach target level with no hypoglycemic events at either setting.

In some aspects, the second processor module comprises a fuzzy logic module operable in response to an event being invoked by a detector module analyzing at least one pattern of glucose levels indicative of at least one event.

In some aspects, the monitoring system comprises an event detector module configured and operable to determine the occurrence or the probability of the patient to be in a special event as a function of a time. The special event may be at least one of sleep, meal, exercise and disease event or rest. The second processor module (e.g. CRM) comprises a plurality of fuzzy engines each being associates with a different special event. The second processor module is configured and operable to alternate between at least two fuzzy logic modules, each handling a different event.

In particular, in some embodiments, the second processor module is operable as a meal detection and treatment module configured and operable to generate an analysis, and if needed a treatment modification, of the patient conditions affected by meal events and therefore to monitor the blood glucose level.

In this case, the input parameters further includes at least one of the following input parameters: time elapsed between detected special events, blood glucose level with respect to the special event.

According to another broad aspect of the present invention, there is also provided a method for automatic monitoring of diabetes-related treatment. The method comprises: obtaining a reference data including individualized patient's profile related data, individualized patient's treatment history related data; analyzing measured data generated by at least one of drug delivery devices and glucose measurement devices; and deciding about treatment modification in accordance with the reference data by controlling the operation of the drug injection devices to enable real-time automatic individualized monitoring of the treatment procedure.

In some aspects, deciding about treatment modification comprises determining the treatment modification in accordance with the individualized patient's treatment history related data.

In some embodiments, analyzing data comprises processing measured data indicative of blood glucose level and generating first processed data indicative thereof, and applying at least one fuzzy logic model to input parameters corresponding to the measured data, the first processed data and the reference data, to produce at least one qualitative output parameter indicative of patient's treatment.

In some embodiments, applying at least one fuzzy logic model to input parameters corresponding to the measured data comprises classifying glucose blood trends in different categories.

In some embodiments, the method comprises applying a prediction model for predicting glucose trend in blood based on the measured glucose level or past glucose level trend.

In some embodiments, deciding about treatment modification comprises at least one of the followings: controlling an individualized basal plan; controlling a insulin/glucagon sensitivity indicative of the correction of the current blood glucose level to a target level, correction of carbohydrates and of the amount of insulin and/or glucagon to be delivered; controlling the individualized blood glucose target level; controlling the insulin and / or glucagon pharmacokinetics settings.

In some aspects, controlling an individualized basal plan comprises obtaining a series of individualized basal treatment rates as a function of time; obtaining the measured data (measured glucose); determining an individualized time delay between a basal treatment rate and changes in the glucose level to thereby obtaining a series of basal treatment rates and corresponding changes in glucose level in a time delay; selecting a basal plan which incorporates the basal rates that minimizes the change in the glucose level in time.

In some embodiments, analyzing data comprises determining occurrence or the probability of the patient being in a special event as a function of time.

According to another broad aspect of the present invention, there is also provided a method for use in automatic monitoring of diabetes-related treatment. The method comprises: analyzing open-loop measured data generated by at least one of drug delivery devices, glucose measurement devices and determining patient's initial treatment profile; receiving continuously measured data generated by at least one of drug delivery devices and glucose measurement devices; applying a self-learning procedure for updating the patient's initial treatment profile during closed loop treatment thereby monitoring of the diabetes-related treatment.

In some embodiments, the patient's initial treatment profile comprises at least one of insulin sensitivity indicative of the correction of the current blood glucose level to a target level, correction of carbohydrates and of the amount of insulin and/ or glucagon to be delivered, basal plan, insulin/glucagon pharmacokinetics associated data, glucose target level or target range level.

In some aspects, determining the insulin sensitivity comprises using at least one of the following parameters: carbohydrate consumed by the patient, measured data, and patient's treatment.

In some aspects, determining patient's initial treatment profile comprises determining the amount of insulin active in the blood.

In some aspects, determining the amount of insulin active in the blood comprises determining the amount as a function of a special event.

According to another broad aspect of the present invention, there is also provided a method for determining insulin basal plan. The method comprises: obtaining a series of basal treatment rates as a function of time; obtaining measured data of glucose level in the patient as a function of time; determining the personal time delay of the patient measured from a basal treatment rates and changes in the glucose level, thereby obtaining a series of basal treatment rates and corresponding changes in glucose level in the patient; and; selecting a basal plan which incorporates the basal rates that minimizes a change in the glucose level in time.

According to another broad aspect of the present invention, there is also provided a method for determining insulin sensitivity for use in close-loop treatment of a patient's need thereof. The method comprises obtaining a first glucose sensor reading and a second glucose sensor reading defining a time window; obtaining the difference between the first and second glucose sensor readings; adjusting the difference between the first and second glucose sensor readings by estimating amount of glucose derived from a consumed carbohydrate within the time window; thereby obtaining an adjusted glucose amount; and determining the insulin sensitivity correction factor in accordance to the relation between the adjusted glucose amount and insulin bolus provided during the time window.

In some aspects, the time window includes an open loop session.

In some aspects, the adjustment is achieved by assuming a coefficient defining the proportion of consumed carbohydrate to glucose derived thereby.

The insulin sensitivity may be modified in accordance with the proportion between minimum sensor reading during the time window and the lowest blood glucose reading recorded in neither during impending hypoglycaemia nor hypoglycaemia. The proportion between minimum sensor reading during the time window and the lowest blood glucose reading recorded in neither during impending hypoglycaemia nor hypoglycaemia can further modified by the maximum sensor reading in a time zone prior to the obtaining of the minimum sensor reading.

## BRIEF DESCRIPTION OF THE DRAWINGS

In order to understand the invention and to see how it may be carried out in practice, embodiments will now be described, by way of non-limiting example only, with reference to the accompanying drawings, in which:

**Fig. 1** is a schematic diagram of a treatment system utilizing a monitoring system of the present invention;

**Fig. 2** is a flow diagram of a method of the present invention for monitoring diabetes treatment of a patient;

**Fig. 3** is a graph illustrating the percentage of insulin active in the blood after a bolus injection;

**Fig. 4** exemplifies the parameters of the fuzzy logic module;

**Fig. 5** is a schematic diagram of a treatment system utilizing a monitoring system of the present invention according to one embodiment of the present invention;

**Fig. 6** is an example of the operation of the monitoring system utilizing a of the present invention;

**Figs. 7A-7D** are a 24 hours closed loop session results conducted on a subject. **Fig. 7A** shows the CGS readings (black line) and the reference measurements (black diamond). **Fig. 7B** shows the insulin treatment delivered by the monitoring system of the present invention. **Figs. 7C** and **7D** show results from control performances comparison between home care (circles) and by using the monitoring system of the present invention (rectangular) using the Control Variability Grid Analysis [9] during time period of 24 hours (Fig. 7C) and during night time (Fig. 7D).

## DETAILED DESCRIPTION OF EMBODIMENTS

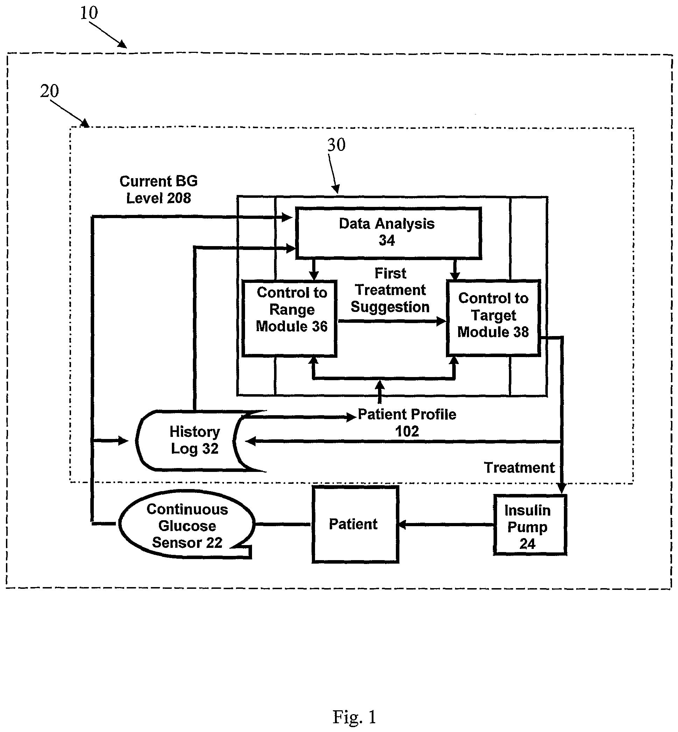

Referring to **Fig. 1,** there is illustrated, by way of a block diagram, a treatment system **10** for carrying out diabetes treatment (controllable delivery of insulin and glucagon), utilizing a monitoring system **20** of the present invention. The monitoring system **20** is associated with a glucose measurement device **22** (continuous glucose sensor), and a drug delivery device **24** (insulin pump). The drug delivery device may also comprise a glucagon delivery pump.

The monitoring system **20** comprises a memory utility **32** (referred in the figure as *History Log*) for storage and/or update of reference data, including individualized patient's profile related data, and individualized patient's treatment history related data. The control unit **30** comprises a first processor module **34** for processing measured data (referred in the figure as *Data Analysis*) indicative of blood glucose level **208** and generating first processed data indicative thereof, a second processor module, that can be also denoted as a control to range module (CRM) **36,** comprising a fuzzy logic module; the fuzzy logic module receives input parameters corresponding to the measured data **208,** the first processed data and the reference data, and processes the received parameters to produce at least one qualitative output parameter indicative of patient's treatment parameters. The control unit **30** is also includes a control to target module (CTM) **38** for final determining whether any of the patients conditions/treatment is to be modified.

Measured blood glucose (BG) level from measurement device 22 (either directly measured or predicted from measured tissue glucose level, as the case may be) enters the control unit **30.**

The second processor **36** receives quantitative input parameters corresponding to the measured data, the first processed data and the reference data, and processes the received quantitative parameters to produce qualitative output parameters indicative of patient's conditions and enabling to determine whether any of these conditions is to be modified. Output of the data analysis module **34** (first processed data) is processed by the fuzzy module of the second processor **36.** The qualitative output parameters of the fuzzy logic module **36** are then processed by a third processor module which can be also denoted as the CTM **38** to determine whether any of the patient's conditions/treatment is to be modified. The final decision relating data from module **38** may be used for updating reference data in the memory utility **32.**

Measured data may also include special event, such as meals, physical activity, sleep time etc.

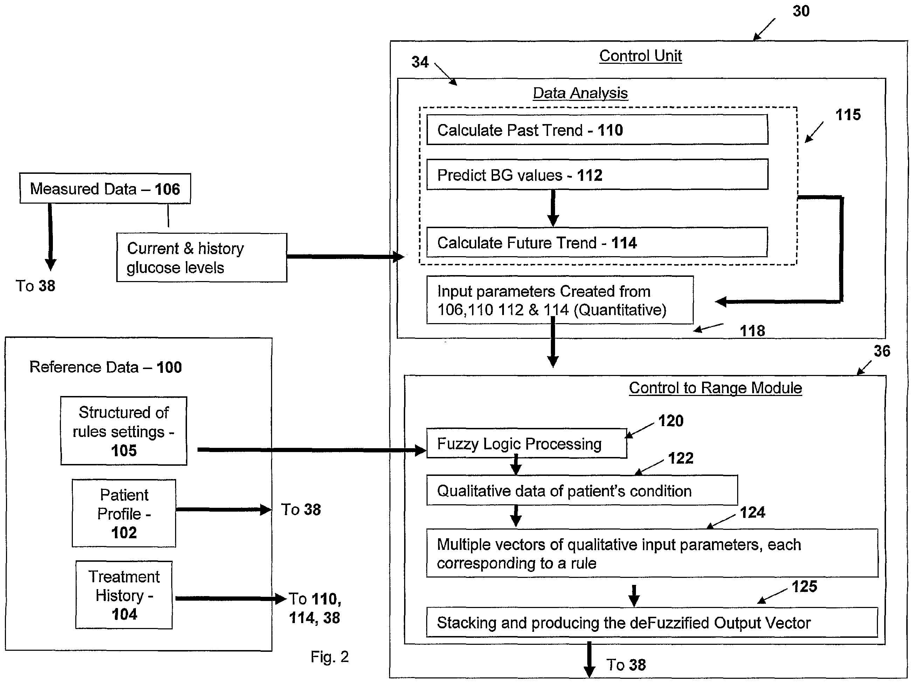

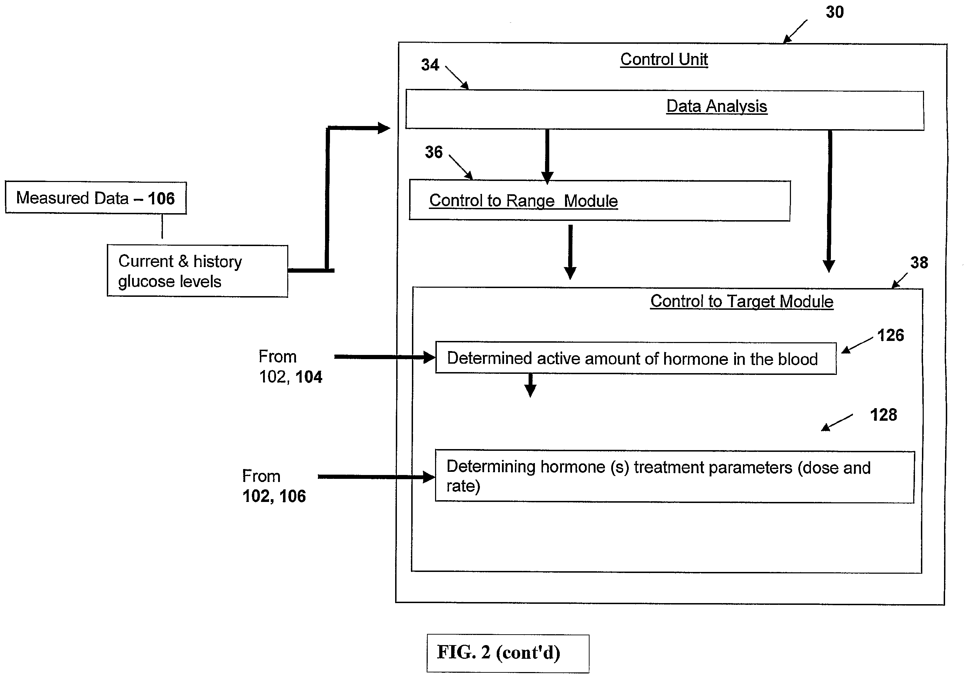

Reference is now made to **Fig. 2** exemplifying a flow diagram of a method of the present invention for automatic monitoring of diabetes-related treatment. Generally, the method comprises analyzing data generated by at least one of drug delivery devices and glucose measurement devices; identifying patient's conditions; and deciding about treatment modification by controlling the operation of the drug injection devices to enable real-time automatic individualized monitoring of the treatment procedure.

In some aspects, analyzing the data comprises providing reference data (step **100**). The reference data includes patient's profile related data **102;** treatment history related data **104,** and a structure of rules or "table of rules" settings **105.** The structure of rules settings are based on the physician approach of evaluating the measurements. The patient's profile related data **102** includes a set of parameters (and calibratable or updatable during the monitoring procedure or during the treatment) about the patient's condition. For example, the patient profile is extracted from collecting data several days prior to connecting the patient to the monitoring system.

In some aspects, the set of parameters is automatically modified by a learning algorithm.

In some aspects, the treatment modification comprises at least one of the followings: controlling an individualized basal plan; controlling patient specific insulin sensitivity for glucose levels (referred as a "correction factor") indicative of the correction of the current blood glucose level to a target level and of the amount of insulin/ and or glucagon to be delivered; controlling the individualized blood glucose target level; controlling the insulin and/or glucagon pharmacokinetics settings to determine the sensitivity of each patient to insulin and/or glucagon respectively.

More specifically, at least one of the followings conditions is controlled:

(1) Basal Plan: The rate of insulin to be injected to the patient during an entire day, according to the time of the day. For example, type 1 patient receives a continuous dose of insulin during the day. This dose can be changed during the day, depending on the change in the patient sensitivity to insulin. Basal Plan can be represented as a series of individualized basal treatment rates as a function of time. The role of the basal treatment is to treat with the endogenic release of glucose by the liver. Therefore, an optimal basal plan will keep the glucose levels stable.

(2) Correction Factor (CF) Insulin/Glucagon Plan: The following equation (1) is used to correct the current BG level to the target level (defined as a reference level for Insulin/glucagon calculation) and to calculate the Insulin/Glucagon bolus:

$$CorrectionBolus \left( Insulin / Glucagon \right) = \frac{abs \left( CurrentBG - T\arg et \right)}{CF} \quad (1)$$

Due to the change insensitivity to Insulin/Glucagon, the CF can be set for each hormone according to the time of the day.

(3) BG Target - The blood glucose level target is defined per patient as a reference level to be used for example for the correction of the Insulin/Glucagon bolus.

(4) Insulin/Glucagon Pharmacokinetics (PK) Settings: A precaution curve is developed to determine the sensitivity of each patient to Insulin/Glucagon, as will be detailed below.

(5) Optionally, the structure of rules settings of the fuzzy logic module such as categorized blood levels (e.g. very low, low, normal, normal high, high and very high) as will be detailed below.

Turning back to **Fig. 2,** the measured data **106** is indicative of the BG level at a certain period of time, being directly measured in the blood or the subcutaneous tissue.

The analyzing of the data is carried out by processing measured data **106** in the data analysis **34** and generating first processed data indicative thereof (step **115**). A fuzzy logic model is applied (step **120**) to quantitative input parameters (step **118**) corresponding to the measured data **106,** the first processed data by using a structure of rules settings to produce qualitative output parameters indicative of patient's conditions.

In some aspects, processing of the measured data (step **115**) includes calculation of a past trend in a glucose level change (step **110**), predict the future BG level value (step **112**), and using the prediction results to calculate a future trend (step **114**).

In this connection, it should be understood that the glucose past/future trend is a parameter influenced by three factors: (*i*) the average rate of change in the glucose level in mg/dl per minute in a certain time window (i.e. the average rate of change), (*ii*) the course of change (i.e. ascending or descending) and (*iii*) the duration of this course.

The quantitative input is a vector of parameters supplied from the measured data relating modules **106,110,112** and **114.**

For example, the quantitative input include the followings four parameters: the past trend, the future trend, the current BG level and the predicted level of the BG.

The fuzzy logic processing **120** is utilized to transform, using the structured of rules settings, the quantitative input vector to qualitative output vector (e.g. multiple vector) (step **122**) denoted as *Fuzzified input vectors* indicative of the patient's condition. In some cases, multiple *Fuzzified input vectors* are obtained from the fuzzy logic processing and each *Fuzzified input vector* is associated with a matching rule (step **124**) of the "table of rules" defined above. In these cases, each matching rule is assigned with a statistical agreement factor which describes to what degree each rule is applied. All applied rules are stacked according to their statistical agreement and a *deFuzzy Function* calculates the *deFuzzified Output Vector* (step **125**) which includes the fuzzy logic recommendation to changes in the treatment in percentages.

For example, the following input vector: [0.7 110 2 170] is interpreted as follows: in the last 20 minutes, the trend was 0.7 [mg/dl/min], the current blood glucose level is 110 [mg/dl], the predicted trend of the blood glucose level is 2 [mg/dl/min] and the predicted value in the 30 minutes is 170 [mg/dl]. When this input vector goes through the fuzzy logic module **36,** it is translated to the following *Fuzzified input vectors:*

1. [High Normal VeryHigh NormalHigh]
2. [High Normal VeryHigh High]

These *Fuzzified Input Vectors* match rule number **73** (73 % agreement) and rule number **204** (27 % agreement). Both of these rules outputs take into consideration and their output member functions be stacked according to their weight (i.e. their statistical agreement percent).

The *deFuzzy Function* calculates the center of weight of those stacked functions (for each of the outputs separately) to weight all the relevant rules and gives the following *deFuzzified Output Vector:* [50 2.59 0]

Generally, each rule includes a modification of the current treatment delivered to the patient, adapted to a specific patient condition indicated by the *Fuzzified input vector.* As described above, the treatment parameters (*i.e.* deFuzzified output vector) include at least one of the following parameters: the modification of the basal rate and/or the insulin/glucagon bolus percentage. Each rule is also associated with a contribution factor (weight) which designates the likelihood of the patient's condition being associated with the specific rule. More specifically, the weight is the probability of such rule to occur in real life, quantized to a number between 0-1. The weight can also be determined in accordance with the importance assigned to the rule. In addition, the weight may also be in accordance with a special event handled by the fuzzy logic engine.

The initial recommendation received from the CRM **34** is in percentage. To determine the dosing amount of the two outputs in units or units/hour, the CTM **36** considers the recommendation of the CRM **34** as well as the glucose target level. Special glucose dynamics analysis is then applied, assuming the dosing regimen history and safety constraints related to the insulin pharmacodynamics, and amount of glucagon and/or insulin active to yield the final dosing recommendation.

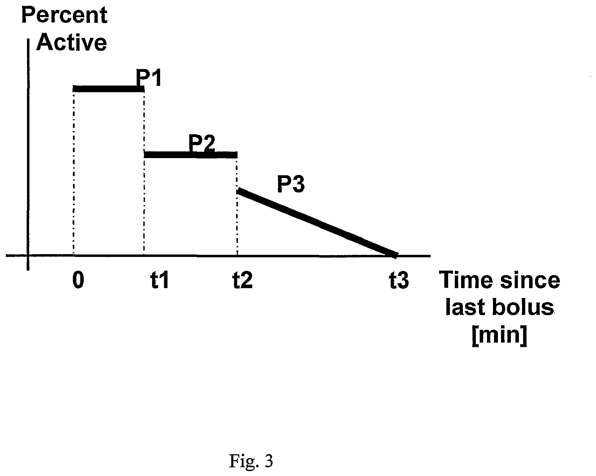

The current amount of glucagon and/or insulin active ($G_{active}$, $I_{active}$) section in the blood is determined according to the patient's profile **102** (step **126**), as exemplified in **Fig. 3,** illustrating the precaution curve determining the pharmacodynamics of a patient to insulin/glucagon. This curve is indicative of the percentage of the insulin/glucagon "active" in the blood at a certain time after the delivery of the insulin/glucagon bolus. The present invention therefore provides a system for use in monitoring diabetes treatment of a patient, the system is configured and operable to modify or provide a treatment (i.e. insulin/glucagons bolus or basal treatment) in accordance to the insulin/glucagons pharmacodynamics of the treated patient. In some aspects, insulin/glucagons pharmacodynamics is represented by a curve or a function describing the percentage (or otherwise amount) of the insulin/glucagon "active" in the blood at a certain time after the delivery of the insulin/glucagon bolus. Moreover, the present invention also provides a method for use in monitoring diabetes treatment of a patient. The method comprises obtaining insulin/glucagons pharmacodynamics of the treated patient; and adjusting a treatment (i.e. insulin/glucagons bolus or basal treatment) in accordance to the insulin/glucagons pharmacodynamics of the treated patient.

The amount of insulin (e.g. percentage) present in the blood is represented at three different period of times (P1, P2, P3) characterizing the activity of the insulin since the last bolus injection. Similar graphs, specific to each patient, designating the patient's absorbance (i.e. decay rates) of insulin/glucagon after bolus or basal treatment, can be generally included in the patient's profile. These decays rates may be used together with the treatment history to determine the amount of active insulin/glucagon present in the blood.

The calculation of the active insulin and active glucagon is done by the CTM module **38** using insulin and glucagon treatment history **104** and the patient's individual pharmacodynamics of glucagon and insulin taken from the patient profile **102,** as detailed above.

The calculation of the active glucagon at the current moment is performed as follows: The times and doses of glucagon are given, denoted as *TG* and *VG,* both vectors of size *N.* The current time is denoted by $t_0$. The active glucagon is denoted by $G_{active}$. The activity function of the glucagon $f_G(t)$ is determined by the patient individual settings:

$$f_G(t) = \begin{cases} P_1 & t \le t_1 \\ P_2 & t_1 < t \le t_2 \\ \dfrac{P_3(t - t_3)}{(t_3 - t_2)} & t_2 < t \le t_3 \\ 0 & t_3 < t \end{cases}$$

Where $t_{1\text{-}3}$, $P_{1\text{-}3}$ are Glucagon time constants which are individually set for each patient, and can be learned and updated automatically by a self-learning algorithm.

The active glucagon is calculated as follows:

$$G_{active} = \sum_{i=1}^{N} VG[i] \cdot f_G(t_0 - TG[i])$$

Similarly, the active insulin can also be calculated at the current moment:

The times and doses of insulin are given, denoted by $TI$ and $VI$, both vectors of size $K$. The current time is denoted by $t_0$. The active insulin is denoted by $I_{active}$.

The activity function of the insulin $f_I(t)$ is determined by the patient individual settings:

$$f_I(t) = \begin{cases} P_4 & t \le t_4 \\ P_5 & t_4 < t \le t_5 \\ \dfrac{P_6(t - t_6)}{(t_6 - t_5)} & t_5 < t \le t_6 \\ 0 & t_6 < t \end{cases}$$

where $t_{4\text{-}6}$, $P_{4\text{-}6}$ are insulin time constants which are individually set for each patient, and can be learned and updated automatically by a learning algorithm.

The active glucagon is calculated as:

$$I_{active} = \sum_{i=1}^{K} VI[i] \cdot f_I(t_0 - TI[i])$$

The amounts of hormones (i.e. insulin and/or glucagon) to be delivered is determined (step **128**) by the CTM module **38** based on the initial recommendation received from the fuzzy logic module **36** (in percentage unit), the patient's treatment history **104**, the insulin/glucagon sensitivity (from the patient profile **102**) and the amount of hormones active in the blood **126**, for example as follows:

The fuzzy logic output vectors are indicative of $G_p$, $B_p$ and $Ba_p$. being the percentage recommendations for the Glucagon, Bolus Insulin and Basal Insulin respectively. ($G_p$ varies from 0 to 100 [%], $B_p$ varies from 0 to 100 [%] and $Ba_p$ varies from -100 to 100 [%]). The corresponding amounts of Glucagon, Bolus Insulin and Basal Insulin to be received by the drug delivery device are denoted as $G_a$, $B_a$ and $Ba_a$. $S$ is the last sensor reading. $CF_G$ and $CP_I$

are the glucagon and bolus insulin sensitivity factors, which are a part of the patient's profile and set individually for each patient and can be learned in real-time. They are time-dependent and change for different times of the days to reflect natural changes in glucagon and/or insulin sensitivity.

*GT* is the patient individual glucose target level.

Basically the amount of glucagon and insulin dose treatment is defined respectively as follows:

$$G_s = \frac{S-GT}{CF_G} * G_p * 0.01 - G_{active},$$

$$B_s = \frac{S-GT}{CF_I} * B_p * 0.01 - I_{active}$$

$G_{active}$, $I_{active}$ being the active glucagon and insulin whose calculation was defined above. If $G_s$ is negative or $G_p$ is lower than 50%, $G_s$ is 0. If $B_s$ is negative, $B_s$ is 0.

Similarly, the basal treatment is defined as follows: $Ba_s = f_{BA}(t_0) * (1 + 0.01 * Ba_p)$, $f_{BA}$ *is the patient's basal* plan indicative of the basal rate for each hour of the day. The function is defined in the patient's profile and can be defined individually for each patient. In addition, this function can be updated by a given data set indicative of the precedent modified treatments using the teachings of the present invention.

Determining the glucagon bolus, basal rate and the bolus treatment, recent treatments are taken into account. $t_G$ and $t_B$ are the time which passed since the last glucagon delivery and the last bolus insulin delivery, respectively. In case, there was no glucagon delivery or no bolus insulin delivery, $t_G = \infty$. and $t_B = \infty$. $t_0$ is the current time. The response time to glucagon/insulin absorption are the constant times $t_G$ determined by the activity time of the glucagon and insulin.

These are individual settings for each patient, for example as follows:

$$\text{If } t_G \leq t_1 \; G_a = G_s, \; B_a = 0 \text{ and } Ba_a = 0$$

$$\text{If } t_1 < t_G \leq t_2 \; G_a = G_s, \; B_a = 0 \text{ and } Ba_a = Ba_s = 0$$

$$\text{If } t_3 < t_G,$$

the following approach has to be adopted: *BT* is the glucose level threshold which allows bolus delivery. *FB* is defined as the first bolus to be delivered typically having a relatively high value. *SB* is defined as the second bolus to be delivered typically having a lower value than *FB.*

*FB* is true if $S > BT$ and $B_s > 0.5$ and $t_B \leq t_4$. Otherwise *FB* is false.

*SB* is true if $S > BT$ and $B_s > 0.25$ and $t_B > t_4$. Otherwise *FB* is false.

If *SB* is true or *FB* is true then $G_a = 0$, $B_a = B_s$ and $Ba_a = Ba_s$.. Otherwise $G_a = 0$, $B_a = 0$ and $Ba_a = Ba_s$.

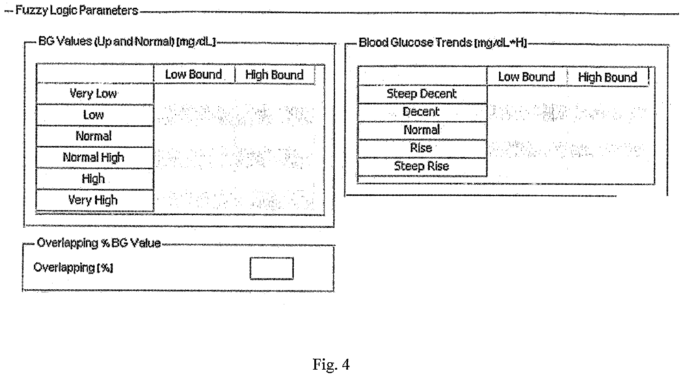

Reference is made to **Fig. 4,** illustrating the qualitative input parameters definition of the fuzzy-logic module **38.** These parameters are individualized (i.e. adaptable to each patient) and they can be automatically changed by the control unit.

For example, the qualitative input parameters include fuzzy values of the BG values in mg/dL categorized in six levels (very low, low, normal, normal high, high and very high) and having a low bound and a high bound. The qualitative input parameters also include fuzzy trend of the BG trends in mg/dL/min categorized in five levels (Steep Descent, Descent, Normal, Rise and Steep Rise).

The first processor module **34** preprocess the measured data **106** to calculate trends in the glucose traces (past trend **110** and future trend **114**) and predict the future glucose trace **114** in a certain horizon.

Trend of glucose level is determined as follows. Trend of glucose level can be determined in accordance with the average rate of change in glucose levels in a certain time window. The average rate of change in glucose level in a certain time window ($Avg \; \dot{G}[t_i]$), for example, can be calculated with a moving average method to determine the amplitude (to quantify the trend) and the course of the trend. The trend of glucose level can be used in turn to select a qualitative input parameter which suitably describes the trend as detailed herein. A trend of glucose level determined with respect to a time zone prior to a present time is denoted as past trend. Therefore, past trend can relate to a trend preceding a contemporary measured glucose level.

The trend duration factor can be employed to provide the trend a time measure of coefficient. The trend duration factor $\tau_{TD}$ can thus be defined as follows:

$$\tau_{TD} = \begin{cases} 1, 0 \le T_{SLTC} \le \tau_1 \\ 2\left(\dfrac{T_{SLTC} - \tau_1}{\tau_2}\right) + 1, \tau_1 < T_{SLTC} \le \tau_3 \quad (1.1) \\ 3, T_{SLTC} > \tau_3 \end{cases}$$

where $T_{SLTC}$ [min] is the point in time when the glucose trend changes from descent to ascent or vice versa, and $\tau_i$ is a time constant. The trend parameter is defined as a function of $Avg\ \dot{G}[t_i]$ and $\tau_{TD}$. For example, the trend parameter can be determined as follows:

$$\text{calculated trend} = Avg\ \dot{G}[t_i] \times \tau_{TD}.$$

For example, if the past BG levels in the past 20 minutes were BG = [153,140,137,128,120], and the time difference between each glucose reading is 5 minutes; the $Avg\ \dot{G}[t_i]$ will be -1.33 mg/dl/min. Since this $Avg\ \dot{G}[t_i]$ has a negative sign, it means the glucose levels are descending. For example, if the $T_{SLTC}$ is 45 minutes (i.e. the glucose levels are descending for 45 minutes) then $\tau_{TD}$ is 2. Thus, the calculated trend will be -2.66 mg/dl/min.

To predict future glucose levels, several prediction models may be used independently or as a combination with the monitoring technique of the present invention. The prediction models enable to overcome sensing and delivery delays. The predictor output is used by the fuzzy logic module.

As indicated above, the CRM 36 uses the reference data **100** and may be a Mamdani-type fuzzy logic controller with four inputs: past and future glucose trend ($\dot{BG}_{Past}$ and $B\tilde{G}_{Future}$) as well as current and future glucose level ($BG_{Curr}$ and $B\tilde{G}_{Future}$). For example, a set of treatment rules was developed, with two outputs for each rule: (a) change in basal rate ($Ba_P$) and (b) portion of insulin bolus ($B_P$) (in percents from the patient's basal plan and the calculated bolus, respectively). To translate the clinical meaning of the input parameters using the fuzzy sets of rules, each member function for the input parameters had to have an interval in which the function's value is 1, followed by a smooth decrease to 0 outside this interval. Therefore, two-sided Gaussian curve member functions were selected. For the output parameters, Gaussian member functions were selected in order to prevent redundancy and to maintain the smooth transition between member functions.

The fuzzy rules were phrased in collaboration with the medical staff. The rules were designed to keep the glucose levels stable within the 80-120 mg/dl range. To evaluate the rule antecedents (i.e. the IF part of the rules), the AND fuzzy operation was used. The output (defuzzification) was calculated by a centroid method. The CRM output treatment suggestion was then transferred to the CTM **38.**

By way of non-limiting examples, the fuzzy logic modules of the present invention can be implemented by using computerized engines such as MATLAB by MathWorks. Where exemplification relates to MATLAB, reference to member function (MF) shall refer to build-in member function provided therein.

The followings inputs are examples of the qualitative parameters that may be used in the fuzzy logic module of the present invention.

**Input 1:** past trend indicative of the calculated trend of the blood glucose level, based on data recorded by the sensor in the past 20 minutes.

**Input 2:** future trend indicative of the calculated trend of the blood glucose level for the next 30 minutes, based on the predicted data.

The past trend and future trend values are classified as follow:

Steep descent - The range is defined from -5 [mg/dl/min] to -2 [mg/dl/min].The member function is defined as a Z-shaped function using the range borders -0.1 / +0.1 respectively as the Z-Shaped function parameters.

Descent - The range is defined from -2 [mg/dl/min] to -0.5 [mg/dl/min].

The member function is defined as a Gauss2 function using the range borders +0.1 / -0.1 respectively and 0.075 as the variance.

<u>Stable -</u> The range is defined from -0.5 [mg/dl/min] to +0.5 [mg/dl/min].

The member function is defined as a Gauss2 function using the range borders +0.1 / -0.1 respectively and 0.075 as the variance.

<u>Rise -</u> The range is defined from +0.5 [mg/dl/min] to +2 [mg/dl/min].

The member function is defined as Gauss2 function using the range borders +0.1 / -0.1 respectively and 0.075 as the variance.

<u>Steep rise -</u> The range is defined from +2 [mg/dl/min] to +5 [mg/dl/min].

The member function is defined as an S-Shaped function using the range borders +0.1/10.1 respectively as the S-Shaped function parameters.

The person skilled in the art would appreciate that the ranges and time interval can also be modified in accordance to a particular treatment to be envisaged.

**Input 3:** current blood glucose level indicative of the last blood glucose level recorded by the sensor.

**Input 4:** future level indicative of the predict blood glucose level in the next 30 minutes.

The current blood glucose level and the future level indicative of the blood glucose level are classified as follow:

<u>Very Low -</u> The range is defined from 50 [mg/dl] to 70 [mg/dl]

The member function is defined as a Z Shaped function.

<u>Low -</u> The range is defined from 70 [mg/dl] to 90 [mg/dl]

The member function is defined as a Gauss2 function.

<u>Normal -</u> The range is defined from 90 [mg/dl] to 140 [mg/dl]

The member function is defined as a Gauss2 function.

<u>Normal High -</u> The range is defined from 140 [mg/dl] to 170 [mg/dl]

The member function is defined as a Gauss2 function.

<u>High -</u> The range is defined from 170 [mg/dl] to 250 [mg/dl]

The member function is defined as a Gauss2 function.

<u>Very High -</u> The range is defined from 250 [mg/dl] to 500 [mg/dl]

The member function is defined as an S Shaped function.

All the parameters (S-Shaped and Z-Shaped functions parameters, Expectancy and Variance for the Gauss2 functions) for the member functions are calculated to meet the following rules: (1) the S-Shaped and Z-Shaped functions have to meet at y=0.5; and (2) S-Shaped and Z-Shaped functions have 5% of overlapping.

The person skilled in the art would appreciate that the ranges and time interval can also be modified in accordance to a particular treatment to be envisaged. The followings outputs are examples of the qualitative output parameters:

**Output 1:** Percentage of change of basal rate i.e. basal rate indicative of the recommended change, in percents relatively to the default contemporary basal rate (0%), in the delivered basal rate. The percent change can be between -100 % (stopping insulin delivery) to 100% (double the default contemporary basal rate). This range can be quantized into equally separated steps.

**Output 2:** Percentage of bolus indicative of the suggested percent of the calculated insulin bolus. The percent change can be between 0 % (No bolus) to 100% (All bolus). This range can be quantized into equally separated steps wise ranges.

**Output 3:** Optionally, glucagon indicative of the suggested percent of the calculated glucagon. The percent change can be between 0 % (No Glucagon) to 100% (All Glucagon). This range can be quantized into equally separated steps wise ranges..

The number of input may be from one to four inputs and the number of outputs may be from one to three outputs.

The structures set of rules can comprise a combination of treatment strategies that can be modified according to each treatment procedure. The strategies may for example overlap while other strategies may be independent from each other. These strategies are represented by a certain relationship between the qualitative input parameters and the corresponding output parameters. The monitoring system of the present invention can determine which appropriate set of rules (appropriate number and combination) can be used to suggest optimal output parameter(s).

For example, the set of rules includes 96 rules, such as:

- **Rule #7:** If the *Current Blood Glucose Level* is *Low* than do not give any bolus;
- **Rule #22:** If *Current Blood Glucose Level* is *Normal* and the *Future Trend of Blood Glucose* is *Descent* than decrease the basal rate by 60%;

- **Rule #28:** If the *Current Blood Glucose Level* is *Normal* than do not change the basal rate;
- **Rule #53:** If the *Current Blood Glucose Level* is *NormalHigh* and the *Predicted Blood Glucose Level* is at *NormalHigh* than increase the basal rate by 60%;
- **Rule #55:** If *Past Trend of Blood Glucose* is *Not Descending,* the *Current Blood Glucose Level* is at *NormalHigh,* the *Future Trend of Blood Glucose* is *Stable* and the *Predicted Blood Glucose Level* is *AboveNormal* than give 50% of the suggested bolus.

Generally, each rule includes a relationship (e.g. modification) between the current specific patient's condition deduced from the values of the input parameters and the appropriate treatment to be delivered to the patient. In particular, the rules can define a relationship between qualitative parameters and a suggested treatment to the patient. For example, the rule can provide relationship between past traces or patterns of glucose measurements to the appropriate treatment. In another example, rule can provide relationship between predicted traces or patterns of glucose measurements to the appropriate treatment. The appropriate treatment can accommodate bringing the range of measured glucose level to a desired range. The patterns or traces (past or predicted) can be represented by a calculated trend. In respect, glucose traces or patterns can be represented by a series of glucose measurements each obtained at a certain time. Thus, glucose traces or patterns can also be represented by at least two glucose measurements obtain at a time interval. Predicted trends can be deduced from the past traces or patterns i.e. past traces or patterns can be used to determine a predicted traces or patterns. Such determination is typically performed by employing a prediction model, some of which are known in the art. Moreover, one element (a glucose level) of a predicted trace or pattern can be selected to be the predicted blood glucose level or a future level.

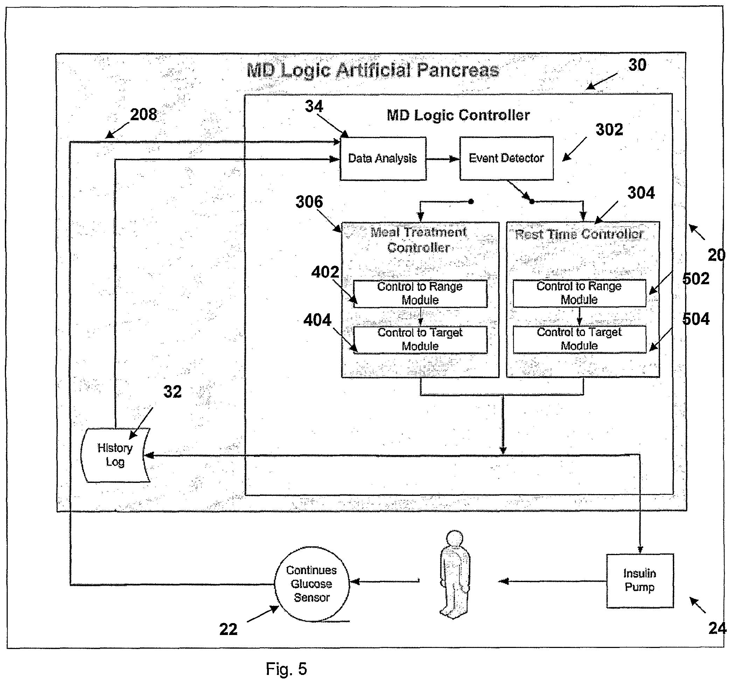

Reference is now made to **Fig. 5** exemplifying a flow diagram of a treatment system utilizing a monitoring system of the present invention according to one embodiment of the present invention.

In some embodiments, the system comprises an event detector module 302 operable to determine the occurrence of an event or the probability of the patient to be in a special event as a function of a time. The special event may be sleep, meal, exercise or disease event. The event detector module is designed to detect such special dynamics associated with each special event. Based on the event that was detected, the proper CRM and CTM are selected.

In some embodiments, at least two controllers are used: rest time controller (for example, the fuzzy logic engine previously discussed above) and a controller designed to deal with the special event, such as a meal, which is referred to as meal treatment module/meal time controller. Therefore, the present invention provides for alternating between at least two fuzzy logic engines (rest time controller and meal time controller).

According to some embodiments of the present invention the control unit **30** comprises an event detector **302** capable for detecting meal events. In case a meal event was detected, a meal treatment module **306** configured and operable to generate an analysis of the meal event is activated. The meal treatment module **306** if needed provides a treatment modification of the patient conditions to suite the meal events. In other cases, when no meal event was detected, the Rest Time Controller **304** is operable. Each controller has its own CRM (**402** and **502**) and CTM (**404** and **504**), respectively. The CRM **502** and CTM **504** of the Rest Time Controller **304** are similar to the modules described above. The CRM **402** of the meal treatment module **306** runs a different table of rules. Each rule can comprise a proposed modification of the possible insulin/glucagon treatment during meal.

Specifically, an event detection module **302** is utilized to detect an event which requires specialized treatment. For example, a meal detection module can be used in order to allow a treatment suitable to an event of meal. This module monitors the blood glucose level and analyzes pattern(s) or traces of glucose levels. In some embodiments, the meal event detector can use the definitions of the glucose qualitative parameters as they were defined for the fuzzy logic module above. On detection of an abnormality in the blood glucose level, a special event is invoked allowing the system and providing the required resources of time (or otherwise) to handle the event.

In addition, a procedure or test can be used to detect the occurrence of a special event such as a meal event. Several tests can be employed in this respect. A test can also be employed to deny a meal event from the patient. In some aspects, a meal event is determined in accordance to a pattern or trace of glucose measurements.

The following terms are used in the followings possible tests:

The term *"Relevant Trend for Special Event Long"* refers to the trend of the blood glucose level log/ pattern as determined in N samples, typically the recent or last N samples. Optionally, the trend can be determined in accordance to method previously elaborated herein. The trend(s) can conveniently be denoted as $a_1 .. a_N$ and the relative times are $\tau_1 .. \tau_N$ while $\tau_i > \tau_{i+1}$.

The term *"Relevant Trend for Special Event Short"* refers to the trend of the blood glucose log/ pattern as determined in M samples while M<N. Typically the recent or last M samples are used in this event. The trend(s) are $a_1 .. a_M$ and the relative times are $\tau_1 .. \tau_M$ while $\tau_i > \tau_{i+1}$. Optionally, the trend is can be determined in accordance to method previously elaborated herein

The term *"Duration"* refers to a predefined number of sample which represents the amount of samples used for analysis.

The term *"Differential for Special Event Long"* refers to the slope (or derivative) of the blood glucose log/ pattern as determined in N samples, typically the recent or last N samples. The trend(s) are $d_1..d_N$ and the corresponding sample times of the trend(s) are $\tau_1.. \tau_N$ while $\tau_i > \tau_{i+1}$.

The term *"Differential for Special Event Short"* refers to the slope (or derivative) of the blood glucose log/ pattern as determined in M samples while M<N. Typically, the recent or last M samples are used in this event. The trends are $d_1.. d_M$ and the corresponding sample times are $\tau_1.. \tau_M$ while $\tau_i > \tau_{i+1}$.

In some aspects, an event is determined in accordance to pattern or traces of glucose level measurements. In some embodiments, occurrence of the event is determined in accordance to a trend of pattern or traces of glucose measurements. The event can be a meal event or a default stable glucose level (i.e. a steady state of measured glucose level). In some embodiments, the trend is any of Relevant Trend for Special Event Long or Relevant Trend for Special Event Short.

Specifically, an event (such as a meal event) can be determined in case the trend exceeds a defined threshold or a threshold of defined qualitative input parameters. Optionally, the event can be determined if the calculated trend exceeds a preceding trend of traces of glucose measurements. In some aspects, an event can be determined if the calculated trend exceeds a defined threshold for a defined duration.

In addition, an event (such as an exercise event) can be determined in case the trend decreases below a defined threshold or a threshold of defined qualitative input parameters. The occurrence of the event can be determined if the calculated trend decreases below a preceding trend of traces of glucose measurements. In some aspects, the event can be determined where the calculated trend decreases below a defined threshold for a defined duration. For example, test A positively identifies a meal event if the following condition is satisfied $\forall a \in$ *Relevant Trend for Special Event Short:*

    i. $a_i \geq a_{i+1}$
    ii. $a_1 \geq w \cdot$ (*Low Boundry of Steep Rise*) + (1- *w*) $\cdot$ (*Low Bound of Rise*) , 0 < *w* < 1.
    where, w is a weight factor which will be set empirically;

The qualitative parameters may be defined as low boundary of steep rise and low bound of rise set empirically by the user or automatically by an automated procedure

Test B will positively detect a meal event if the following conditions are satisfied $\forall a \in$ *Relevant Trend for Special Event Long:*

    iii. At least *for Duration* of the samples $a_1 \geq$ *Stable*
    iv. $a_1 >$ *Stable* and $a_2 >$ *Stable*
    where, the definition of *Stable* can be according to the definition of *Stable* member functions in the fuzzy engine or otherwise set by the user.
    Test C will positive detect a meal event if the following terms are satisfied $\forall a \in$ *Relevant Trend for Special Event Short:* The difference between the blood glucose level at $\tau_1$ and the blood glucose level at $\tau_M$ is at least X
    **v.** The difference between the blood glucose level at $\tau_1$ and the blood glucose level at $\tau_N$ is at least Y, while Y $\geq$ X

Test D will positively identify a meal event if the following terms are satisfied $\forall a_i | i =$ **1,2,3,** $a_i > w \cdot$ (*Low Boundry of Steep Rise*) + (1- *w*) $\cdot$ (*Low Bound of Rise*) , where, *w* is a weight factor which will be set empirically or by automated procedure.

Test E checks that slope of the last (or previous) sample is smaller than *Low Bound Rise.* Test E can be used to deny a meal possibility from the patient.

At each testing point during the operation of the systems or method disclosed herein, one or more of the above tests above may be satisfied. Other test can also be devised in that respect. A meal/event detection module can be configured and operable to detect an event such as a meal by performing the detection tests. By running the meal detection module on a large set of measured data, the probability of each single test to detect the meal/event (i.e. the test's positive predictive value) can be ascertained, as well as the probability combination of tests to detect the meal/event at the same sample time. In addition, conditional probability of single test and/or combination of test(s) to detect the meal/event given a previous sample can be ascertained. The meal detection module can be tested on empirically data in order to calculate each test's positive predictive value. The result of the calculation can then be used as the probability for each test to positively detect a meal event. The absence of a meal event can also be detected in similar manner.

The following table provides an example for the probabilities of each test (that were described above) and tests combinations that were calculated using the 10 adult group from the training version of the UVa/Padova simulator [5]. The test or test combination frequency of use (1 - most frequently used and 14 - rarely used) is a parameter which scales the tests according to the number of times in which they were activated. For example, the probability of Test A to positively detect a meal is 100% however it is rarely activated.

| Test Combination | Probability to positively detect a meal event | Test's frequency of use |
|---|---|---|
| A | 100% | 13 |
| AB | 88% | 12 |
| ABC | 72% | 4 |
| ABCD | 90% | 1 |
| ABD | 0% | 14 |
| AC | 84% | 5 |
| ACD | 100% | 10 |
| AD | 0% | 14 |
| B | 23% | 8 |
| BC | 28% | 6 |
| BCD | 72% | 2 |
| BD | 54% | 9 |
| C | 43% | 3 |
| CD | 83% | 11 |
| D | 67% | 7 |

The output of the meal detection module can be either positive or negative. In addition, the output of the meal detection module will be the probability that a special event, i.e. meal or sudden rise of the blood glucose levels, occurs.

A threshold probability (P%) can be determined for the occurrence of the special event. Once the system recognizes that the probably for a special event exceeded the determined threshold, it can switch the CRM and CTM previously used i.e. either a default CRM and CTM (referred in figure 5 as Rest Time Controller **304**) or another treatment module designed for other special events.

The CRM **402** of the meal treatment module **306** uses a fuzzy logic engine which typically has the same working principles described for the rest time CRM **502**. It may differ in the input parameters and it may have the same output parameters or modified output parameters. A possible strategy for meal related CRM fuzzy logic engine ("special event fuzzy engine") is based on the time elapsed from the first detected special event of a measured series. It can thus allow application of treatment rules comprising greater amount of insulin in a first stage in order to deal with the special event. On the other hand, it allows the system to be more decisive on decreasing the basal rate and even stopping the insulin infusion in order to prevent hypoglycemia.

There are several conditions which can control the switching or alternating between the meal treatment module **306** and Rest time controller **304.**

For example, if the last used module is the rest time controller, the conditions can be as follows:

1. Obtaining the blood glucose level reading;
2. If the probably of special event is P% or higher, switching to the special event fuzzy engine, otherwise continue with the rest time controller

For example, if the last used controller is the meal treatment module:

1. Get the blood glucose level ($[BG_{i-N} : BG_i]$) reading and past glucose trend $\left( \left[ \dot{BG}_{i-N} : \dot{BG}_i \right] \right)$ for time samples $[t_{i-N} : t_i]$;

2. if one of the following conditions is satisfied- switching to the rest time controller;

a. If each value $\left[ \dot{BG}_{i-N} : \dot{BG}_i \right]$ is the range of *Stable* AND each of the samples $[BG_{i-N} : BG_i]$ is lower than

a threshold, for example, 130 mg/dl;

b. If each of the samples [$BG_{i-N}$ : $BG_i$] is in the blood glucose range of [Blood Glucose Target - Y%, Blood Glucose Target + Z%] AND each of the samples $\left[ \overset{\square}{BG}_{i-N} : \overset{\square}{BG}_i \right]$ is lower than high boundary of the *Stable* range;

3. Otherwise, if there has been more than T minutes from the first detected special event of the previous/ last series and at current sample, a special event was detected as well; set the current sample as the first detected special event of a new series and continue using the meal treatment module;

4. If none of the above conditions is satisfied, use meal treatment module;

The input parameters for the special event fuzzy engine are as follows: Blood glucose level trend in the last $T_1$ minutes, current blood glucose level, predicted blood glucose level trend in the next $T_2$ minutes, predicted blood glucose level in $T_2$ minutes, time elapsed since a first detected special event of a previous/ last measurement series, blood glucose level trend in the last $T_3$ minutes before the first detected special event of the previous/ last series and blood glucose level at the time of the first detected special event of the last series.

The output parameters for the special event fuzzy engine are as follows: change of basal infusion rate from the default basal and percent of insulin/glucagon bolus.

By way of non-limiting example, the input parameters and the corresponding membership functions used herein below refer to MATLAB built membership functions as follows: *"smf",* shaped membership function; *"Zmf",* Z-shaped membership function; *"gauss2mf",* Gaussian combination membership function; *"trimf",* Triangular-shaped built-in membership function; and *"trapmf",* Triangular-shaped built-in membership function.

Qualitative inputs parameters:

• Past Trend of Blood Glucose (i.e. Blood glucose level trend in the last $T_1$ minutes [mg/dl/min])

| MF name | MF function | MF ranges |
|---|---|---|
| Rapid Descent | Zmf | -5,-2.5 |
| Moderate Descent | gauss2mf | -2.5,-1.5 |
| Slow Descent | gauss2mf | -1.5,-0.5 |
| Stable | gauss2mf | 0.5,0.5 |
| Slow Increase | gauss2mf | 0.5,1.5 |
| Rapid Increase | Smf | 2.5,5 |
| Slow Increase or Stable | gauss2mf | 0,1.5 |
| Some Descent | Zmf | -5,-0.5 |
| Not Rapid Descent | gauss2mf | -2.5,0 |
| Not Rapid Increase | gauss2mf | 0.5,2.5 |

• Current Blood Glucose level [mg/dl]

| MF name | MF function | MF ranges |
|---|---|---|
| Low and Below | Zmf | 20,70 |
| Normal | gauss2mf | 90,150 |
| High | gauss2mf | 150,220 |
| Very High | Smf | 220,300 |
| Below Normal | Zmf | 20,90 |

(continued)

| MF name | MF function | MF ranges |
|---|---|---|
| Above Normal | Smf | 130,300 |
| High and Above | Smf | 180,300 |

• Future Trend of Blood Glucose (i.e. Predicted blood glucose level trend in the next $T_2$ minutes [mg/dl/min])

| MF name | MF function | MF ranges |
|---|---|---|
| Rapid Decrease | zmf | -5,-2.5 |
| Slow Decrease | gauss2mf | -1.5,-0.5 |
| Stable | gauss2mf | -0.5,0.5 |
| Slow Increase | gauss2mf | 0.5,1.5 |
| Moderate Increase | gauss2mf | 1.5,2.5 |
| Rapid Increase | smf | 2.5,5 |
| Some Decrease | zmf | -0.5,-5 |
| Not Rapid Decrease | gauss2mf | -0.5,-2.5 |
| Not Increasing | zmf | -5,0.5 |
| Some Increase | smf | 0.5,5 |
| Not Rapid Rise | gauss2mf | 0.5,2.5 |
| Not Slow Rise | smf | 1.5,5 |

• Predicted blood glucose level in $T_2$ minutes [mg/dl]

| MF name | MF function | MF ranges |
|---|---|---|
| Low and Below | zmf | 20,90 |
| Normal | gauss2mf | 90,140 |
| High | gauss2mf | 180,220 |
| Very High | smf | 220,300 |
| Not Low | smf | 110,180 |
| Below Normal | zmf | 20,90 |
| Not Above Normal | zmf | 70,130 |
| Above Normal | smf | 130,180 |
| High or Very High | smf | 180,300 |

• Time past since the first detected special event of the last series [min])

| MF name | MF function | MF ranges |
|---|---|---|
| Meal Start | zmf | 0,45 |
| During Meal | smf | 45,300 |

• Blood glucose level trend in the last $T_3$ minutes before the first detected special event of the last series [mg/dl/min])

| MF name | MF function | MF ranges |
|---|---|---|
| slow Increase | gauss2mf | 0.5,1.5 |
| Moderate Increase | gauss2mf | 1.5,2.5 |
| Rapid Increase | smf | 2.5,5 |
| Not Slow Rise | smf | 1.5,5 |
| Some Increase | smf | 0.5,5 |
| Not Rapid Rise | gauss2mf | 0.5,2.5 |

• Blood glucose level at the time of the first detected special event of the last series. [mg/dl]

| MF name | MF function | MF ranges |
|---|---|---|
| Low and Below | zmf | 20,70 |
| Normal | gauss2mf | 90,130 |
| Very High | gauss2mf | 220,300 |
| Below Normal | zmf | 60,95 |
| Above Normal | smf | 135,220 |

Output parameters:

• Change in percent of basal infusion rate from the default basal [%]

| MF name | MF function | MF ranges |
|---|---|---|
| 0 | trapmf | -100 |
| 0.2 | trimf | -80 |
| 0.5 | trimf | -50 |
| 1 | trimf | 0 |
| 1.5 | trimf | +50 |
| 2 | trapmf | +100 |

• Percent of bolus [%]

| MF name | MF function | MF ranges |
|---|---|---|
| 0 | trapmf | 0 |
| 0.5 | trimf | 50 |
| 1 | trimf | 100 |
| 1.2 | trimf | 120 |
| 1.35 | trimf | 135 |
| 1.7 | trimf | 170 |
| 2 | trimf | 200 |
| 2.5 | trimf | 250 |
| 3 | trapmf | 300 |

The person skilled in the art would appreciate that the glucose ranges, member functions and time intervals can also be modified in accordance to suit particular treatment envisaged.

The table of rules of the special event module (or special event CRM) may have a number of inputs from one to seven inputs and a number of outputs from one to two. The ranges of such inputs and outputs are defined *per se* and are not different for each fuzzy logic module.

For example, the CRM for meal event includes 130 rules. Some exemplary rules are provided as follows:

- Rule #21: If *Time Passed from Meal Start* is not greater than 45 minutes, *Current Blood Glucose Level* is *Normal* and *Predicted Blood Glucose Level* is *Very High* than give 200% of basal and 300% of recommended bolus;
- **Rule #84:** If *Time Passed from Meal Start* is greater than 45 minutes, the *Past Trend of Blood Glucose* is *not increasing rapidly* and *Current Blood Glucose Level* is *High* than give 100% of basal rate and 100% of recommended bolus;
- **Rule #110:** If *Time Passed from Meal Start* is greater than 45 minutes, *Current Blood Glucose Level* is *High,* the *Future Trend of Blood Glucose* is *not increasing rapidly* and *Predicted Blood Glucose Level* is *High* than give 100% of basal rate and 120% of recommended bolus;
- **Rule #126:** If *Time Passed from Meal Start* is greater than 45 minutes, the *Past Trend of Blood Glucose* is *not Descending Rapidly, Current Blood Glucose Level* is *Above Normal* and *Predicted Blood Glucose Level* is *Above Normal* than give 100% of basal and do not give any bolus;
- **Rule #128:** If Time Passed from Meal Start is greater than 45 minutes, the Past Trend of Blood Glucose is Stable, Current Blood Glucose Level is Above Normal and Predicted Blood Glucose Level is Above Normal than give 100% of basal and 100% of recommended bolus.

The meal detection and treatment module uses a combination of fuzzy logic model and trend analysis of glucose profile. The system including a meal detection and treatment module was evaluated on 24 hour *in silico* trials with three meals using the UVA/Padova simulator. The improved system succeeded to increase the time spent between 70-180mg/dl by 10% (p=0.02) by decreasing the time spent above 180mg/dl in similar percent (p=0.02) and without increasing time spent below 70mg/dl. In both systems, time spent below 70mg/dl was on average less than 1.6%. In addition, mean BG level was decreased from 150 mg/dl to 138mg/dl (p=0.002).

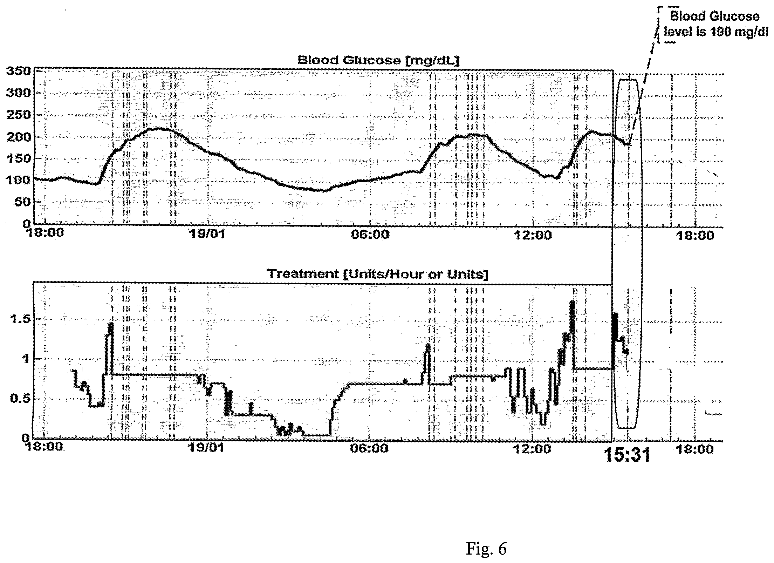

Reference is made to **Fig. 6** illustrating an example of the operation of the system of the present invention. The encircled area is the current decision point (15h31) of the system at which the measured data is a glucose level of 190 mg/dl. The portion of the graph before the encircled area is the measured data stored in the *History Log.*

The patient profile includes basal plan, correction factor, active insulin etc. For example the basal rate taken from the basal plan assigned for the time 15h31 is 0.9 units per hour, the correction factor is 50 mg/dl/unit and the predefined glucose target level is 110 mg/dl.

The data analysis **34** provides for example that the past trend is 0 mg/dl/min, the future trend is -0.24 mg/dl/min and the predicted glucose level is in the coming 30 minutes is 179 mg/dl/min.

Since no special event was detected the Rest Time controller is applied.

The CRM **402** uses appropriate rules from the table of rules therefore increasing the basal rate by 79% and deliver 73% of the calculated bolus. The CTM **404** outputs that for a glucose level of 190 mg/dl, the insulin amount 1.6 units. 73% of the 1.6 units of recommended bolus are 1.1 units. The suggestion may also be stored in the History Log.

Since a bolus is recommended, the CTM decides to ignore the CRM recommendation of increasing the basal rate and sends the following command to the delivery pump: basal rate = 0.9 units/hour and bolus units. The insulin pump **24** receives the amount of insulin to be delivered.

According to another broad aspect of the present invention, there is provided a method which improves and maintains the closed-loop system performance and therefore the treatment on a specific patient. The method is a learning algorithm for automatic analysis of control performances against intra-patient variances in the glucose/insulin dynamics, with adjustments of the control parameters accordingly. The learning method can be performed by an independent module to extract the patient profile from data.

The method comprises analyzing initial settings based on open loop data, as well as making periodical adjustments during close-loop operation.

The performances of the learning integrated method were evaluated using ten subject adult population from the UVa/Padova simulator. A nominal simulation day consists with three meals (at 9am, 2pm and 7pm, of 40g, 70g and 50g, respectively) was defined. All subjects followed the same scenario which includes open-loop un-perfect meals carbohydrate estimation (2 days) followed by close-loop (5 days) therapy. The learning method was automatically activated after the open-loop section as well as after every 24h of close-loop until achieving optimal performances. The clinical measures achieved during optimal day of close-loop (OpCL), day1 of close-loop (D1CL) and average open-loop (AOL) were compared (one way ANOVA). BG below 70mg/dl was 0-0.4% in all days of simulation. While there was no significant change in the administrated insulin, time spent in 80-120mg/dl was significant greater in OpCL ($53\pm8\%$)

versus D1CL (41±8%) and AOL (18±8%) (p<0.001). Mean BG was 121±5mg/dl in OpCL compared to 129±7mg/dl in D1CL (p=0.01) and 140±7mg/dl in AOL (p<0.001).

The present invention discloses an automated learning method and systems for permitting automatic determination of the patient's initial treatment profile. These methods can be performed by a dedicated module configured and operable to execute them. The learning method can be divided into two main sub-procedures:

I) An initial learning, which receives measured data of the subject during open-loop associated treatment. Typically, the measured data is collected while the patient is performing his own treatment at home. The data is typically generated by at least one of drug delivery devices and glucose measurement devices and comprises the sensor readings, meal amounts and times and/ or insulin treatment(s), either bolus and basal. The initial learning procedure can analyze the data (measured or calculated) and determine automatically the patient's initial treatment profile. The patient's initial treatment profile include at least one of correction factor, basal plan, insulin/glucagon pharmacokinetics associated data, glucose target level or target range level, glucagon dosage, insulin bolus and insulin activity model;

II) The continuous learning procedure can update the patient's treatment profile during the closed-loop operation. The patient's treatment profile include at least one of basal plan, insulin sensitivity factors for carbohydrates and glucose level correction, glucagon sensitivity factor and insulin/glucagon pharmacodynamics associated data. The patient's treatment profile can be adaptive in accordance with closed-loop history log.

The initial learning sub procedure and the continuous learning procedure can be performed separately, sequentially or in combination.

In some aspects, the insulin sensitivity factors (for carbohydrates and glucose level correction, denoted as CF) are determined during the initial learning procedure. In some aspects, the insulin sensitivity factor is determined at least in accordance with carbohydrate consumed by the patient, measured data of glucose sensor reading, and the patient's treatment which can include insulin dosage, or basal plan.

Optionally, the data is collected while the patient was at home. In one example, optionally during the initial learning procedure, an insulin sensitivity factor $CF_1$ is determined as follows:

- Determining $CF_1$ in accordance with carbohydrate amount, glucose and insulin related data:

$$CF_1 = \frac{G_e - G_s + dC \cdot C}{B}$$

wherein $G_e$ is the first sensor reading [mg/dl] of the open loop session; $G_s$ is the last sensor reading [mg/dl] of the open loop session; $dC$ is a glucose to carbohydrate ratio. The ratio of glucose to carbohydrate can be 3.33, (based on empirical knowledge); C is amount of carbohydrate consumed [e.g. gr] during the open loop; and B is the amount of bolus insulin provided [units of insulin] during the open loop session.

$G_e$ - $G_s$ is defined as the difference between $G_e$ (a first glucose sensor reading) and $G_s$ (a second glucose sensor reading). The time interval between the two glucose sensor readings can be defining a time window.

In some aspects, the glucose derived from the consumed carbohydrate within the time window is estimated. Such estimation can be performed by obtaining an amount of carbohydrate consumed in the time window and transforming the carbohydrate amount to glucose derived thereof.

The transformation can be performed by determining a coefficient defining the proportion of consumed carbohydrate to glucose derived thereby e.g. ($dC$ above). By multiplying the coefficient with the amount of carbohydrate consumed in the time window, the glucose derived from the consumed carbohydrate is determined.

Adjustment of difference between the first and second glucose sensor reading can be effected by summing the difference between the first and second glucose sensor readings and the glucose derived from the consumed carbohydrate; thereby obtaining an adjusted glucose amount.

Determining the insulin sensitivity (e.g. CF1) can be determined in accordance to the relation between the adjusted glucose amount and insulin bolus provided during the time window. Relation can be the defined by the proportions between the respective values as shown above.

In some aspects, $G_e$ may be the first reading of a portion of an open loop session and/ or $G_s$ may be the last sensor reading of a portion of an open loop session. In some embodiments, $G_e$ may be the first reading of a portion of a closed loop session and/ or $G_s$ may be the last sensor reading of a portion of a closed loop session.

Optionally, the sensitivity factor such as $CF_1$ may be modified based on analysis of the quality of glucose control of the patient using the data that was collected while the patient was at home.

In some aspects, insulin sensitivity factor (e.g. *CF1*) is modified in accordance with measured glucose levels. For example, insulin sensitivity factor is modified in accordance with minimum sensor reading or lowest blood glucose reading recorded in neither during hypoglycaemia nor hypoglycaemia. In a specific example, the insulin sensitivity is modified in accordance with proportion between minimum sensor reading during the time window and the lowest blood glucose reading recorded in neither during hypoglycaemia nor hypoglycaemia. In some embodiments, the insulin sensitivity is modified in accordance to the maximum sensor reading in a time interval prior to the obtaining of the minimum sensor reading (an example is shown below).

Therefore, insulin sensitivity or *CF1* can further be modified in accordance with factor *(a)* to produce a modified correction factor $CF_2$ in accordance with the formula: $CF_2 = a \cdot CF_1$ wherein factor *(a)* is defined as the factor of modification of $CF_1$.

Factor a may be determined, according to the following procedure:

$$\text{If } \mathit{Thypo} > 0 \text{ or } \mathit{Tihypo} > 1$$
$$\text{If } (\mathit{Speak} > \mathit{Smin}) \text{ and } (\mathit{Speak} > \mathit{UpperLimit})$$
$$\mathrm{a} = (\mathit{Speak} - \mathit{Smin}) / (\mathit{Speak} - \mathit{UpperLimit});$$
$$\text{Else}$$
$$\mathrm{a} = \mathit{UpperLimit}/\mathit{Smin};$$
$$\text{End}$$
$$\text{Else}$$

wherein **Thypo** is a percent of time spent in defined hypoglycemia range during the relevant period; **Tihypo** is a percent of time spent in defined impending hypoglycemia range during the relevant period; **Smin** is a minimum sensor reading during the relevant period; **Smean** is the average sensor readings during the relevant period; **Smax** is a maximum sensor reading during the relevant period; **Speak** is a maximum sensor level in time range of up to three hours before the *Smin* tim, during the relevant period; **UpperLimit** is the lowest blood glucose reading that is recorded neither during impending hypoglycemia nor hypoglycemia; **Sn_low** is the lower boundary of "strict normal" glucose range (can be empirically defined as the glucose range in the range of about 80-120 mg/dl), which is typically set to be 80; **Sn_high** is the higher boundary of "strict normal" glucose range, which can be set to be 120; **dN** is the subtraction **Sn_high-Sn_low**.

A histogram (or alternatively a distribution function) can be determined by using the measured glucose levels of the patient. The histogram is a function representing occurrences of each measured glucose level of the patient during a certain time window. P can be defined as summation of the occurrences (or an accumulated measured glucose levels) at an interval of a specific width (*dN* representing glucose measurement interval), wherein v is the initial glucose reading in this specific window, individualized for each patient.

$val = \arg\max_v \{P(v, v + dN)\}$, where $P(v, v + dN)$ is the percentage of glucose readings with the range $[v, v + dN]$ ; $\mathrm{argmax}_v$ means determining the v where P reaches maximum value.

$$a = 0.57 \cdot a\_Tsn + 0.28 \cdot a\_Hyper + 0.15 \cdot a\_Mean \text{ ,}$$

where

$$\boldsymbol{a\_Tsn} = sn\_low/val;$$

$$\boldsymbol{a\_Hyper} = 180/Smax;$$

typically defined empirically

$$\boldsymbol{a\_Mean} = 110/Smean;$$

typically defined empirically

$$W = [0.57 \quad 0.28 \quad 0.15],$$

a weighing vector/coefficients, typically defined empirically.
End

The person skilled in the art would appreciate that the weighing vector can be adjusted or modified to suit particular insulin/glucagons treatments.

In some therefore a histogram representing the occurrence of measured glucose level of the patient during a certain time window is determined. The local maximum (or peak) in a glucose measurement interval can then be obtained, for example by maximizing the function $P(v, v + dN)$ as exemplified above.

Therefore, in some aspects, the insulin sensitivity factor is modified in accordance with the local maximum (or peak) of measured glucose level histogram within a glucose level interval. In some embodiments, the insulin sensitivity factor is modified in accordance the accumulated measured glucose level in the histogram within a glucose level interval. Modification of the insulin sensitivity factor can take the form of transforming the accumulated measured glucose levels in accordance with a weighing vector or coefficient.

In some aspects, the safety of $CF_2$ or $CF_1$ can be tested to verify that whether the insulin dosing provided is safe. The test can be performed by processing a series of glucose sensor reading previously obtained for a treated patient (such as the treated patient) i.e. a previous glucose trace. Thus, sensor readings from the open loop session can be used to simulate insulin bolus recommendations for the closed-loop session.

In some aspects, the test is defined as follows:

If *Bsim > Btotal*

$$CF = \frac{Bsim}{Btotal} \cdot CF_2$$

Else

$$CF = CF_2$$

End

wherein ***Bsim*** is total insulin boluses given by simulated closed-loop system (in case when simulating the open loop sensor readings), ***Btotal*** is the total amount of bolus insulin given during the open loop session.

As described above, the insulin sensitivity can include two separate factors: insulin sensitivity for carbohydrates and insulin sensitivity for glucose levels correction.

In some aspects, insulin/glucagons pharmacodynamics of an individual is represented by a series or a curve describing the insulin/glucagon "active" in the blood at a certain time associated with a meal event. Therefore, the initial settings can further include determination of the pharmacodynamics parameters for insulin (denoted as active insulin) for the individual patient, as concluded from the open loop data. Active insulin can be defined with reference to a specific meal or to a series of meals.

*Ali* is defined as the active insulin for a specific meal. The time of the meal is denoted as ***T0***. For each meal (carbohydrates consumption noted in open loop data), a first time window is defined starting from the specific meal ***T0*** at the open loop data until the next meal time or until seven hours after the meal, the earlier between the two. Peak sensor value after the meal is identified is denoted as ***Smmax***. Minimum sensor value which occurred after the peak is denoted as ***Smmin.*** The respective time tag when the peaks where obtained is typically recorded, defining a second time window between the time ***Smmax*** and ***Smmin.*** Sensor data during the second time window is obtained. The obtained sensor data can be represented by a series of [***Ti, Vi***]*,* where ***Ti*** are the time tags of sensor readings with reference to the beginning the meal ***T0,*** and ***Vi*** are sensor values measured at their respective ***Ti***

In some aspects, the measured sensor reading is normalized. The measured sensor reading can be normalized to value between 0 and 1. *Ni* represents the normalized value of the respective ***Vi***.

***Ni*** can be calculated as follows:

*Ni = Vi*/(*Smmax-Smmin*).
Normalized series **[*Ti, Ni*]** can thus be obtained.

In some aspects, the series (either **[*Ti, Vi*]** or **[Ti, Ni])** are modified (or "forced") into a monotonic series such as a monotonic non-increasing series. Thus, in one embodiment, a non-increasing series is obtained by associating each *Ni* to a minimum normalized *Nj,* j=1 to i.

In other words, Ni = min({Nj}, j=1:i).

For example, for the series $N_j$={1,0.9,0.8,1.2,0.7}, $N_i$ will be {1,0.9,0.8,0.8,0.7}.

The meal peak value i.e. at *T0,* can be added [T0, 1] at the beginning of the series **[*Ti, Ni*]**.

The series thus obtained represents the active insulin *Ali* for a specific meal.

Therefore, the present invention provides a method for determining a series or a curve describing the insulin/glucagon in the blood at a certain time window associated with a meal event, the method comprises obtaining plurality of sensor data measured during the time window starting at T0, representing the time of the occurrence of the meal; optionally normalizing the sensor data; and transforming the measured sensor data (or normalized sensor data) to a monotonic non-increasing series or curve; thereby obtaining a series or a curve describing the insulin/glucagon in the blood at the time window associated with the meal event.

The method for determining a series or a curve describing the insulin/glucagon in the blood can be performed either during open-loop session or during a closed loop session (i.e. in real time). According, the patient's treatment profile can be modified before, at an initial learning phase or during treatment.

In some aspects, the plurality of sensor data measured during the time window can be represented by a series of [*Ti, Vi*]*,* where *Ti* are the time tags of sensor readings with reference to the beginning the meal *T0,* and *Vi* are sensor values measured at their respective *Ti.*

In some aspects, the step of transforming the measured sensor data to a monotonic non-increasing series comprises associating each *Vi* of the resultant monotonic non-increasing series to a minimum *Vj,* j=1 to I in the measured sensor data.

In some aspects, the step of transforming the normalized measured sensor data to a monotonic non-increasing series comprises associating each *Ni* of the resultant monotonic non-increasing series to a minimum normalized *Nj,* j=1 to I in the normalized sensor data.

Where more than one meal took place, the active insulin series for a set of meals can be obtained. In one embodiment, the active insulin for a set of meals is the median of all the meal series {*Ali*}. The resultant series, denoted as *AI_total* represents an active insulin curve. The values represent the percentage of insulin which is still active in the treated patient. For example, elements of [t=25, v=0.8], within the *AI_total* series, can indicate that 25 minutes after injecting a bolus, 80% percent of insulin was still active.

In some aspects, basal plan is monitored and optionally modified. Insulin basal rate typically affects the dynamics of the glucose levels, but this effect is subtle compared to the observed effect of carbohydrates consumption (meals) and given insulin (boluses). Therefore, the open loop data is "cleaned" by taking out every segment of glucose levels that might be affected by meals or bolus insulin.

In some aspects, an effect window or zone of both meal and/ or bolus injection is determined (either automatically or manually such as by the physician). For example, the effect zone, can be three hours measured from the giving of the bolus or the meal. Optionally, the effect zone is set to 2, 3.5, 4, 6 or 8 hours measured from the giving of the bolus or the meal, or even more.

Glucose sensor readings (*G(t)*) and the basal rates (*B(t)*) during the effect zone can be referred to as "clean data". A change of glucose levels in time (t) can be defined by: *DG(t) = dG/dt.*

Basal rates at *B(t)* will affect *DG(t+A)* due to the delay time caused by infusing. A, the time delay can be derived by determining *A=argmax(A, E{B(t)DG(t+A)})*, wherein *A* is the parameter which maximizes the expectancy of the multiplied series *B(t)*DG(t+A).*

With a given *A,* a series of *[DG(t+A), B(t)]* can be defined. Therefore, in some embodiments, the relationship between bolus injections and change of glucose level is represented by the series *[DG(t+A), B(t)],* thereby obtaining a series of basal treatment rates and corresponding changes in glucose level in a treated patient. Optionally, the series *[DG(t+A), B(t)]* can be interpolated the series values to find *B(t)* when *DG(t+A)* = 0, thereby enabling a selection of a basal treatment rate which minimizes a change in the glucose level (e.g. *B(t)*) from the series of basal treatment rates. The obtained basal treatment rate can be used to modify the basal plan of the treated patient e.g. by inserting the obtained basal treatment rate as an element in the basal plan. Thus, the basal treatment plan obtained provides for minimal changes in glucose level. This method can be used for controlling a personal basal plan of the patient.

Therefore, in one of its aspects, the present invention relates to a method for determining insulin basal plan suitable for a patient in need thereof, the basal plan is characterized by reducing the changes to the glucose levels in the treated patient. The insulin basal plan is derived from a series of basal treatment rates. The basal plan obtained can thus be optimal. The method can be performed either during open-loop or closed-loop sessions.

The method for determining of insulin basal plan from a series of basal treatment rates for a patient in need thereof, comprises: obtaining a series of basal treatment rates as a function of time; obtaining measured data of glucose level in the patient as a function of time; determining series of changes in glucose levels as a function of time; determining the personal time delay of the treated patient which is estimated from the series of basal treatment rates and the series of changes in glucose levels, thereby obtaining a series of basal treatment rates and corresponding changes in glucose level in the patient; selecting a basal plan which incorporates the basal rates that minimizes the change in the glucose level.

In some aspects, measured data of glucose level in the patient is derived from glucose sensor readings, denoted as *G(t))* above. In some embodiments, basal treatment rates as a function of time is derived from basal rates, denoted as *B(t)* above.

In some aspects, the method is applied during a predefined effect zone. In some embodiments, a change of glucose levels in time (t) can be defined by: *DG(t)* = *dG/dt*.

In some aspects, the personal time delay of the treated patient is determined by maximizing the expectancy of the multiplied series $B(t)*DG(t+A)$ such that **A=argmax(A, E{B(t)DG(t+A)}),** wherein *A* is the parameter which maximizes the expectancy of the multiplied series $B(t)*DG(t+A)$.

In some aspects, the continuous learning procedure (or Runtime learning) modifies the insulin sensitivity factor (*e.g. CF*) according to the observable/measured data. The insulin sensitivity factor can be modified in accordance with at least one of the set{*CF(i), LOG(i)*}, where *CF(i=1)* is the first CF and *LOG(i=1)* is the relevant *LOG* for the corresponding period of *CF(i=1),* i.e. the time zone in which the system utilized *CF(i).*

The first step of the continuous learning procedure is to determine the factor *a* in accordance to the last *CF* and *LOG* in the set. These are denoted for convenience as *CF(END)* and *LOG(END)*. LOG(END) defining the corresponding time zone/ period in which the system utilized *CF(END) .* Factor *a* can be determined as previously noted with respect to initial learning procedure

The modified correction factor **CFnew** can be determined as follows: **CFnew** = *a*CF(END).* In some embodiments, the modified correction factor is verified as reasonable or as safe. Verification of the modified correction factor can be performed by forcing constraints. For example, two constrains change the modified $CF_{new}$ where constraints are not met. The constrains can include two boundaries.

The two constrains are:

1. If *CFnew> UP_Boundary* then *CFnew = UP_Boundary.*
2. If *CFnew< DOWN_Boundary* then *CFnew = DownBoundary.*

where *UP_Boundary and DOWN_Boundary* can be defined as follows:

**UP_Bouhdary** is defined as the smallest *CF* in {*CF(i), LOG(i)*} in which the minimum sensor level reached in the relevant *LOG(i)* was above a certain threshold, for example 70 mg/dl.

**DOWN_Boundary** can be defined according to the following:

The largest CF which caused minimum sensor value below 50 is defined to be *CF1* with minimum sensor level *LEV1.*

The smallest CF which caused minimum sensor value above 50 is defined to be *CF2* with minimum sensor level *LEV2.*

If both CFs exists and *CF1<CF2,* the lower boundary is defined as:

$$DOWN\_Boundary = (70-LEV1)/(LEV2-LEV1)*(CF2-CF1)+CF1.$$

The following is the results of clinical trials using the monitoring system and method of the present invention:

The study group consisted of 7 patients, 5 female and 2 male, aged 19-30 years. Mean duration of diabetes was $10\pm4$ years; mean HbA1C, $6.6\pm0.7\%$; and mean body mass index, $22\pm2.5$ kg/m$^2$. The patients' demographic data, diabetes history, and other significant medical history were recorded, in addition to height, weight, and HbA1c level. The patients wore a CGS (Freestyle Navigator™, Abbott Diabetes Care, Alameda, CA, USA or STS-Seven® System, DexCom, San Diego, CA, USA) and recorded their meals and physical activities for 3-5 consecutive days. These data and corresponding insulin doses (downloaded from the insulin pump) were used to formulate the patient's treatment history for application in the monitoring system of the present invention.

Short-acting insulin analogue (NovoRapid®, Novo Nordisk, Bagsvaerd, Denmark) was used in the clinical trials. The CGS readings were entered (automatically or manually) into the monitoring system of the present invention every five minutes, and the system provided an insulin dose recommendation after each entry.

The control-to-range was set at 90-140 mg/dl, and the control-to-target, at 110 mg/dl. Each clinical session was supervised by a diabetologist who had to approve any treatment recommendation before it was automatically or manually delivered by the pump to the patient. Reference blood glucose levels were measured by the YSI 2300 STAT Plus (YSI, USA) every 30 minutes. Carbohydrate was administered when the reference blood glucose level dropped below 70 mg/dl.

8-hour closed-loop sessions were conducted in the resting state under two conditions: fasting or meal. The subject's insulin pump was replaced by the research insulin pump (OmniPod Insulin Management System™, Insulet Corp, Bedford, MA, USA or MiniMed Paradigm® 722 Insulin Pump, Medtronic, Northridge, CA, USA). In the fasting closed-loop condition, subjects arrived to the clinic in the morning (usually 08h00) after an overnight fast and were instructed to measure their blood glucose at wake up (usually 06h30). If the level was below 120 mg/dl with no hypoglycemia, they were asked to eat 1-2 slices of bread. In the closed-loop sessions with meal challenge, patients arrived to the clinic after about an 8 hours' fast and consumed a mixed meal with a carbohydrate content of 40-60 gr.

Two 24-hour closed-loop visits were conducted. Subjects arrived to the clinic in the afternoon after a fast of at least 3 hours. The subject's insulin pump was replaced with a modified OmniPod insulin pump which has communication abilities to a regular PC. Three standard mixed meals were consumed at 19h30, 08h00 and 13h00, based on the patient's regular diet. The estimated carbohydrate content for each meal was 17.5 to 70 gr. Each patient slept for 7-8 hours at night during the study.

To examine the control performances of the monitoring system of the present invention during the 8-hour closed-loop sessions, two parameters were analyzed: glucose excursion and degree of stabilization.

Glucose excursion is determined by the peak postprandial glucose level and the time from initiation of closed-loop control to return of the glucose level to below 180 mg/dl.

Stable glucose levels were defined as a change of +/- 10 mg/dl for a period of at least 30 minutes. The time from initiation of closed-loop control or mealtime until the stable state was attained, and the average glucose level at the stable state, were calculated.

In addition, 24-hour closed-loop control and the patient's home open-loop control were compared. The percent of glucose readings within, above, and below the range of 70-180 mg/dl was determined. The data set of the open-loop control included sensor readings from the 3-day period prior to the 24-hour closed-loop session. Control variability grid analysis (CVGA) [9] served as an auxiliary outcome measure. In this analysis, the open-loop data set included sensor readings from a period of 9-16 days. CVGA was performed over two time periods: 24 hours and night-time (00h00-08h00).

During all of the experiments, diabetes physicians approved each and every one of the monitoring system of the present invention treatment suggestions.

Reference is made to **Table 1** summarizing the average and ranges results of the 8-hours closed loop sessions clinical studies.

| | Average | Range |
| --- | --- | --- |
| Fasting sessions | | |
| BG at beginning of closed loop session [mg/dL] | 237 | 178 - 300 |
| Time to below 180mg/dL from system connection [hour] | 2.13 | 0.5 - 4.43 |
| Time to stable BG levels [hours] | 4.4 | 2.3 - 6.75 |
| BG level at stabilization [mg/dL] | 112 | 77 - 155 |
| Meal sessions | | |
| BG at beginning of closed loop session [mg/dL] | 96 | 70 - 138 |
| Peak Post prandial BG level [mg/dL] | 234 | 211 - 251 |
| Time to below 180mg/dL from meal onset [hours] | 2.56 | 2.18 - 3 |
| Time to stable BG levels [hours] | 3.43 | 3 - 4.3 |
| BG level at stabilization [mg/dL] | 102 | 70 - 134.5 |

A total of nine closed-loop control sessions were conducted under fasting conditions at rest with six subjects. The average blood glucose level was 237 mg/dl at initiation of closed-loop control and decreased to 106 mg/dl within 4.4 hours. There were no hypoglycemic episodes.

During one of the fasting session, the monitoring system of the present invention succeeded to prevent a hypoglycemic episode after an overdose of insulin was delivered by the patient before his arrival to the clinic. The monitoring system of the present invention detected the overall trend in the patient's glucose level, took the overdose into account, and then decreased the insulin basal rate to full stop. This action successfully lowered the patient's glucose levels to a stable average of 80 mg/dl within 2 hours.

Three meal-challenge sessions were conducted with two subjects. The meal was detected and treated by the module 23 minutes on average after meal consumption. Peak postprandial glucose levels were 234 mg/dl on average, with a maximum of 251 mg/dl (see Table 1). Blood glucose levels, for the meal-challenge sessions, decreased to below 180 mg/dl within 2.5 hours on average and stabilized in the normal range within 3.5 hours for at least one hour.

Two 24-hour closed-loop sessions were conducted subjects #1 (Female, age 30 yr, BMI 22.9 kg/m$^2$, HbA1c 5.9% with 19 years of diabetes duration) and #2 (Male, age 23 yr, BMI 21.2 kg/m$^2$, HbA1c 7% with 8 years of diabetes duration). During the night, blood glucose levels ranged between 80 and 160 mg/dl, with a nadir of 93 mg/dl for subject #1 and 80 mg/dl for subject #2.

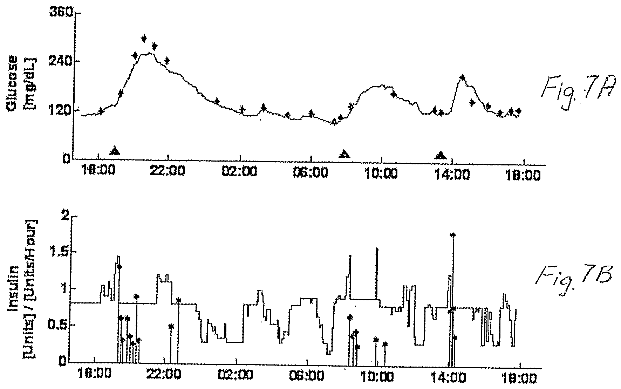

Reference is made to **Figs. 7A-7D** illustrating a 24-hour closed-loop session with subject #1. Glucose levels peaked at 260 mg/dl after dinner, 190 mg/dl after breakfast and 210 mg/dl after lunch. The corresponding values for subject #2 were 221 mg/dl, 211 mg/dl and 219 mg/dl. Between meals, glucose levels returned to below 180 mg/dl within a mean of $2.7\pm0.8$ hours for both subjects. Mean peak postprandial glucose level for overall sessions (8- and 24-hour) was $224\pm22$ mg/dl, and glucose level returned to below 180 mg/dl at a mean interval of $2.6\pm0.6$ hours. Mean time to stabilization was $4\pm1$ hours.

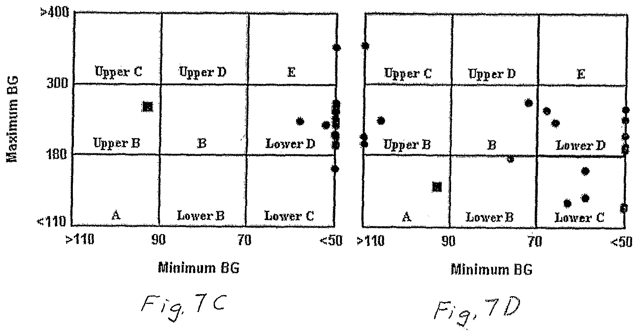

**Fig. 7A** shows the glucose trace including CGS readings (black line) reference measurements (black diamond) and the meal times (black triangles).. **Fig. 7B** shows the insulin treatment (the horizontal lines represent the basal rate, vertical lines with dark circles represent insulin boluses line - basal rate and stem - insulin boluses) delivered by the monitoring system of the present invention during the 24-hour closed-loop trial with subject #1. Results from control performances comparison between home care (circles) and the monitoring system of the present invention (rectangular) using the Control Variability Grid Analysis (CVGA) [9] are shown on **Fig. 7C** (time period of 24 hours) and **Fig. 7D** during night time. **Fig. 7C** shows a control variability grid analysis (CVGA) over a time period of 24 hours for subject #1. **Fig. 7D** shows a control variability grid analysis overnight (00:00-08:00) for subject #1. The nine zones of the CVGA are associated with different qualities of glycemic regulation: A - accurate control, Lower B - benign deviations into hypoglycemia, B - benign control deviations, Upper B - benign deviations into hyperglycemia, Lower C - over correction of hypoglycemia, Upper C - over correction of hyperglycemia, Lower D - failure to deal with hypoglycemia, Upper D - failure to deal with hyperglycemia, and E - erroneous control. In both figures, the circles represent the minimum/maximum glucose level taken from the relevant time period glucose readings during home care and the rectangles indicate the levels during the closed-loop session regulated by using the monitoring system of the present invention.

Based on the control performances analysis, glucose control was found to be better during the 24 hours closed-loop sessions regulated by using the method of the present invention than the pre-study home care.

Seventy-three percent of the sensor values measured 70-180 mg/dl during closed-loop control compared to 70.5% over the 3-day open-loop period prior to the trial day. In addition, none of the sensor readings were below 70 mg/dl during closed-loop control compared to 15.3% for open-loop control. However, 27% of the sensor values were above 180 mg/dl during closed-loop control compared to 14.2% during open-loop control. On CVGA, the monitoring system was maintained benign control over a 24-hour perspective whereas the subjects at home care overcorrected and failed to manage hypoglycemia. During the night as well, the monitoring system maintained benign or accurate control, whereas home care was characterized by great variability. The analysis results for subject #1 are presented in **Figs. 7C-7D.**

As illustrated in **Fig. 7C** and **7D,** CVGA was used to compare the performance of the monitoring system and home open-loop control. The results showed that during open-loop control, there was at least one recording of glucose below 60 mg/dl per day for both subject #1 and subject #2 (**Fig. 7C**). In general, these values appeared after daytime meals, indicating poor postprandial control of glucose excursions. Although only two 24-hour closed-loop experiments were conducted, CVGA revealed a great improvement with the monitoring system during the day and night (**Fig. 7C** and **7D**). Whereas peak postprandial glucose values were similar in both systems, only the monitoring system prevented late postprandial hypoglycemia.

No events of hypoglycemia occurred during either the 8-hour or 24-hour closed-loop sessions. On two occasions (8-hour closed-loop sessions), an impending hypoglycemic event was detected, with glucose levels ranging between 62-65 mg/dl for about 10 minutes. Although the subjects did not experience any symptoms of hypoglycemia, our physician decided to administer 15 gr of fast carbohydrate for safety reasons.

Feasibility studies were conducted in seven adults with type 1 diabetes (age, 19-30 yr; mean diabetes duration, $10\pm4$ yr; mean HbA1C, $6.6\pm0.7\%$). All underwent 14 full closed-loop control sessions of 8 hours (fasting and meal state) and 24 hours.

The mean peak postprandial (overall sessions) glucose level was 224±22 mg/dl. Postprandial glucose levels returned to below 180 mg/dl within 2.6±0.6 hours and remained stable in the normal range for at least one hour. During 24-hour closed-loop control, 73% of the sensor values ranged between 70-180 and mg/dl, 27% were above 180 mg/dl, and none were below 70 mg/dl. There were no events of symptomatic hypoglycemia during any of the trials.

Glucose levels were maintained in the near normal range (80-160 mg/dl) at night. The monitoring system prevented nocturnal hypoglycemia by detecting the overall descending trend in the patient's glucose level and then decreasing the insulin basal rate to full stop. In 2 of the 14 closed-loop sessions, there was a short incident of impending asymptomatic hypoglycemia. The subjects had experienced a symptomatic nocturnal hypoglycemia event (below 50mg/dl) prior to the clinic day, which was treated at home. The monitoring system made reasonable treatment suggestions, which were approved by the diabetes physician in charge, and responded to the descending trend of glucose by lowering the patient's basal rate to full stop. The physician considered the increase in the risk of recurrent hypoglycemia and therefore stopped the experiment.

**Claims**

1. A system for automatic monitoring of diabetes-related treatment of a patient, the system (20) including a control unit (30) which comprises:

   a first processor module (34) for analyzing measured data (106) indicative of blood glucose level (208) and generating first processed data including quantitative parameters indicative of at least past and future trends in the blood glucose level;
   a meal detection module (302) analyzing the first processed data to determine the occurrence of a meal event or the probability of the patient being in a meal event as a function of time in accordance with a pattern of glucose levels;
   a second processor module (36), configured to receive input parameters corresponding to the measured data, said first processed data, and reference data including a structure of rules settings, the second processor module comprising two fuzzy logic modules (402, 502), wherein said second processor module is configured and operable to alternate between said two fuzzy logic modules,
   wherein one of the two fuzzy logic modules is associated with a meal event and the other of the two fuzzy logic modules is associated with a rest event, each of the two fuzzy logic modules being configured to apply to the first processed data and reference data a respective set of rules associated with the respective event so as to produce for said event at least one output parameter for indicating at least one of basal insulin, bolus insulin and bolus glucagon treatment parameters to be modified, wherein the fuzzy logic module associated with a meal event is operable if a meal event was determined by the meal detection module, and else the fuzzy logic module associated with a rest event is operable, and
   a third processor module (38) being configured and operable to receive said at least one output parameter of one of the two fuzzy logic modules, individualized patient's treatment history related data, insulin/glucagon sensitivity from individualized patient's profile related data and a current amount of glucagon and/or insulin active in the blood, and to process said at least one output parameter and said data to determine the amount of dosing of insulin and/or glucagon to be delivered so as to bring the patient's glucose level to a specific target level.

2. A system according to Claim 1, wherein input parameters received by the second processor module further include at least one of current blood glucose level and future blood glucose level parameters.

3. A system according to Claim 1 or 2, wherein said at least one fuzzy logic module comprises a set of rules associated with contribution factors designating the likelihood of a patient condition being associated with a specific said rule, and at least one fuzzy engine utilizing one or more member functions modeled for translating the quantitative input parameters into said at least one output parameter; said second processor module thereby providing control to range output treatment suggestion based on the at least one output parameter of the fuzzy logic module.

4. A system according to any one of the preceding claims, wherein said third processor is configured and operable to determine said modification in accordance with at least one of a glucose target of the patient's profile, patient's insulin or glucagon pharmacodynamics, and said measured data.

5. A system according to any one of the preceding claims, **characterized by** at least one of the following: (1) said control unit is associated with a drug injection device and is configured and operable to control the operation of said drug injection device; and (2) the control unit is operable to update and/or calibrate said individualized patient's profile related data during treatment or during a monitoring procedure.

6. A system according to any one of the preceding claims, comprising a data transceiver for at least one of:

    receiving at least one of said reference data and said measured data; and
    transmitting said at least one output parameter of the control unit to a drug injection device.

7. A system according to any one of the preceding claims, wherein said individualized patient profile related data comprises parameters selected from at least one of insulin sensitivity, glucagon sensitivity, basal plan, insulin/glucagon pharmacokinetics associated data, glucose target level or target range level, and insulin/glucagon activity model.

8. A system according to any one of the preceding claims, wherein said individualized patient treatment history related data comprises a patient insulin delivery regimen given to the patient at different hours of the day.

9. A system according to Claim 1, wherein the input parameters received by the second processor further include at least one of the following: time elapsed between detected meal events and blood glucose level with respect to said meal event.

10. A system according to any one of the preceding claims, wherein said measured data is obtained at a certain time, said measured data comprising at least one of current and past glucose levels relative to said certain time.

11. A method of automatic monitoring of diabetes-related treatment of a patient using a control system which is configured to carry out the steps of:

    at a first processor module (34), analyzing measured data (106) indicative of blood glucose level (208) and generating first processed data including quantitative parameters indicative of at least past and future trends in the blood glucose level;
    at a meal detection module (302), analyzing the first processed data to determine the occurrence of a meal event or the probability of the patient being in a meal event as a function of time in accordance with a pattern of glucose levels;
    at a second processor module (36) comprising two fuzzy logic modules (402, 502), receiving input parameters corresponding to the measured data, said first processed data, and reference data including a structure of rules settings, and alternating between said two fuzzy logic modules;
    at the two fuzzy logic modules, applying to the first processed data and reference data a respective set of rules associated with the respective event so as to produce for said event at least one output parameter for indicating at least one of basal insulin, bolus insulin and bolus glucagon treatment parameters to be modified, wherein one of the two fuzzy logic modules is associated with a meal event and the other of the two fuzzy logic modules is associated with a rest event, and wherein the fuzzy logic module associated with a meal event is operable if a meal event was determined by the meal detection module, and else the fuzzy logic module associated with a rest event is operable; and
    at a third processor module (38), receiving said at least one output parameter of one of the two fuzzy logic modules, individualized patient's treatment history related data, insulin/glucagon sensitivity from individualized patient's profile related data and a current amount of glucagon and/or insulin active in the blood, and processing said at least one output parameter and said data to determine the amount of dosing of insulin and/or glucagon to be delivered so as to bring the patient's glucose level to a specific target level.

12. A method according to Claim 11, wherein said operating each fuzzy logic module comprises at least one of the following:

    classifying glucose blood trends in different categories;
    determining the probability of the patient to be in one of said plurality of events as a function of time,; and
    applying a prediction model for predicting glucose trend in blood based on the measured glucose level.

**Patentansprüche**

1. System zur automatischen Überwachung der diabetesbedingten Behandlung eines Patienten, wobei das System (20) eine Steuereinheit (30) einschließt, die die nachstehendes Bestandteile umfasst:

   ein erstes Prozessormodul (34) zum Analysieren vom Messdaten (106), die den Blutzuckerspiegel (208) anzeigen, und zum Erzeugen erster verarbeiteter Daten, die quantitative Parameter einschließen, die mindestens vergangene und zukünftige Tendenzen im Blutzuckerspiegel anzeigen;
   ein Mahlzeitnachweismodul (302), das die ersten verarbeiteten Daten analysiert, um das Auftreten eines Mahlzeit-Ereignisses oder die Wahrscheinlichkeit, dass der Patient sich in einem Mahlzeit-Ereignis befindet, als Funktion der Zeit in Übereinstimmung mit einem Muster von Glukosewerten ermittelt;
   ein zweites Prozessormodul (36), gestaltet, um Eingabeparameter zu empfangen, die den Messdaten entsprechen, wobei die ersten verarbeiteten Daten, und Referenzdaten, eine Struktur von Regeleinstellungen einschließen, das zweite Prozessormodul zwei Fuzzy-Logik-Module (402, 502) umfasst, wobei das zweite Prozessormodul gestaltet ist und betrieben werden kann, um zwischen den zwei Fuzzy-Logik-Modulen zu wechseln, wobei eines der zwei Fuzzylogik-Module mit einem Mahlzeit-Ereignis verbunden ist und das andere der zwei Fuzzylogik-Module mit einem Ruhe-Ereignis verbunden ist, wobei jedes der zwei Fuzzylogik-Module gestaltet ist, um auf die ersten verarbeiteten Daten und Referenzdaten einen entsprechenden Satz an Regeln anzuwenden, die mit dem jeweiligen Ereignis verbunden sind, um für das Ereignis mindestens einen Ausgabeparameter zum Anzeigen von zu verändernden Basalinsulin-, Bolusinsulin-, Bolus-Glucagon-Behandlungsparametern zu erzeugen, wobei das mit einem Mahlzeit-Ereignis verbundene Fuzzylogik-Modul betrieben werden kann, wenn ein Mahlzeit-Ereignis durch das Mahlzeitnachweismodul ermittelt wurde, und anderenfalls das Fuzzylogik-Modul, das mit einem Ruhe-Ereignis verbunden ist, betrieben werden kann, und
   ein drittes Prozessor Modul (38), das konfiguriert ist und betrieben werden kann, um den mindestens einen Ausgabeparameter von einem der zwei Fuzzylogik-Module, individualisierte, sich auf die Behandlungsgeschichte des Patienten beziehende Daten, Insulin/Glucagon-Empfindlichkeit von individualisierten, sich auf das Patientenprofil beziehenden Daten und die aktuelle Menge an Glucagon und/oder Insulin, die in dem Blut aktiv ist, zu empfangen, und den mindestens einen Ausgabeparameter und diese Daten zu verarbeiten, um die zuzuführende Dosiermenge an Insulin und/oder Glucagon zu bestimmen, um so den Glucosewert des Patienten auf einen spezifischen Zielwert zu bringen.

2. System nach Anspruch 1, wobei durch das zweite Prozessormodul empfangene Eingabeparameter des Weiteren den aktuellen Blutzuckerspiegel und/oder zukünftige Blutzuckerspiegel-Parameter einschließen.

3. System nach Anspruch 1 oder 2, wobei das mindestens eine Fuzzylogikmodul einen Satz an Regeln, die mit Beitragsfaktoren verbunden sind, die die Wahrscheinlichkeit eines Patientenzustandes kennzeichnen, der mit einer bestimmten Regel dieser Regeln verbunden ist, und mindestens eine Fuzzy-Funktionseinheit umfasst, die eine oder mehrere Mitgliederfunktionen nutzt, die zur Überführung der quantitativen Eingabeparameter in den mindestens einen Ausgabeparameter modelliert wurden, wobei das zweite Prozessormodul dadurch eine Steuerung bereitstellt, dass es einen AusgabeBehandlungsvorschlag auf Grundlage des mindestens einen Ausgabeparameter des Fuzzylogikmoduls klassifiziert.

4. System nach einem der vorstehenden Ansprüche, wobei der dritte Prozessor gestaltet ist und betrieben werden kann, um die Modifikation in Übereinstimmung mit dem Glucoseziel des Profils des Patienten und/oder der Insulin- oder Glucagon-Pharmakodynamik des Patienten und/oder den Messdaten zu bestimmen.

5. System nach einem der vorstehenden Ansprüche,
   **gekennzeichnet durch**
   mindestens einen der nachstehenden Punkte: (1) die Steuereinheit ist mit einer Arzneimittel-Injektionsvorrichtung verbunden und ist gestaltet und kann betrieben werden, um den Betrieb der Arzneimittel-Injektionsvorrichtung zu steuern; und (2) die Steuereinheit kann betrieben werden, um die individualisierten, mit dem Profil des Patienten in Beziehung stehenden Daten während der Behandlung oder während eines Überwachungsvorgangs zu aktualisieren und/oder zu kalibrieren.

6. System nach einem der vorstehenden Ansprüche, umfassend einen Daten-Sendeempfänger für:

   Empfangen der Referenzdaten und/oder der Messdaten, und/oder

Senden des mindestens einen Ausgabeparameters der Steuereinheit zu einer Arzneimittel-Injektionsvorrichtung.

7. System nach einem der vorstehenden Ansprüche, wobei die individualisierten, mit dem Patientenprofil in Bezug stehenden Daten Parameter umfassen, ausgewählt aus Insulinempfindlichkeit und/oder Glucagon-Empfindlichkeit und/oder Basalplan, und/oder mit der Insulin/Glucagon-Pharmakokinetik verbundenen Daten und/oder Glucosezielwert oder -zielbereichswert und/oder Insulin/Glucagon-Aktivitätsmodell.

8. System nach einem der vorstehenden Ansprüche, wobei die individualisierten, in Bezug zur Patientenbehandlungsgeschichte stehenden Daten ein Patienten-Insulinzuführungs-Regime umfassen, das dem Patienten an verschiedenen Stunden des Tages verabreicht wird.

9. System nach Anspruch 1, wobei die Eingabeparameter, die durch den zweiten Prozessor empfangen werden, des Weiteren die Zeit, die zwischen ermittelten Mahlzeit-Ereignissen verstrichen ist, und/oder den Blutzuckerspiegel in Bezug auf das Mahlzeitereignis einschließen.

10. System nach einem der vorstehenden Ansprüche, wobei die Messdaten zu einer bestimmten Zeit erhalten werden, wobei die Messdaten aktuelle und/oder vergangene Glucosewerte, bezogen auf die bestimmte Zeit, umfassen.

11. Verfahren zur automatischen Überwachung der diabetesbedingten Behandlung eines Patienten unter Anwendung eines Steuerungssystems, das gestaltet ist, um die nachstehenden Schritte auszuführen:

an einem erstes Prozessormodul (34), Analysieren von Messdaten (106), die den Blutzuckerspiegel (208) anzeigen, und Erzeugen erster verarbeiteter Daten, die quantitative Parameter einschließen, die mindestens vergangene und zukünftige Tendenzen in dem Blutzuckerspiegel anzeigen;
an einem Mahlzeitnachweismodul (302), Analysieren der ersten verarbeiteten Daten, um das Auftreten eines Mahlzeit-Ereignisses oder die Wahrscheinlichkeit, dass der Patient
sich in einem Mahlzeit-Ereignis befindet, als Funktion der Zeit in Übereinstimmung mit einem Muster von Glukosewerten zu ermitteln;
an einem zweiten Prozessormodul (36), das zwei Fuzzylogikmodule (402, 502) umfasst, Empfangen von Eingabeparametern, die den Messdaten entsprechen, wobei die ersten verarbeiteten Daten und Referenzdaten eine Struktur von Regel-Einstellungen einschließen, und Wechseln zwischen den beiden Fuzzylogikmodulen;
an den zwei Fuzzylogik-Modulen, Anwenden eines entsprechenden Satzes an Regeln, die mit dem jeweiligen Ereignis verbunden sind, auf die ersten verarbeiteten Daten und Referenzdaten, um für das Ereignis mindestens einen Ausgabeparameter zum Anzeigen von zu verändernden Basalinsulin-, Bolusinsulin-, Bolus-Glucagon-Behandlungsparametern zu erzeugen, wobei eines der zwei Fuzzylogik-Module mit einem Mahlzeit-Ereignis verbunden ist, und das andere der zwei Fuzzylogik-Module mit einem Ruhe-Ereignis verbunden ist, und wobei das Fuzzylogik-Modul, das mit einem Mahlzeit-Ereignis verbunden ist, betrieben werden kann, wenn ein Mahlzeit-Ereignis mittels des Mahlzeitnachweismoduls ermittelt wurde, und anderenfalls das Fuzzylogik-Modul, das mit einem Ruhe-Ereignis verbunden ist, betrieben werden kann; und
an einem dritten Prozessor-Modul (38), Empfangen des mindestens einen Ausgabeparameters von einem der zwei Fuzzylogik-Modulen, von individualisierten, sich auf die Behandlungsgeschichte des Patienten beziehende Daten, von Insulin/Glucagon-Empfindlichkeit von individualisierten, sich auf Patientenprofile beziehende Daten, und von der aktuelle Menge von Glucagon und/oder Insulin, die in dem Blut aktiv ist, und Verarbeiten des mindestens einen Ausgabeparameters und der Daten, um die zuzuführende Dosiermenge an Insulin und/oder Glucagon zu ermitteln, um so den Glucosewert des Patienten auf einen spezifischen Zielwert zu bringen.

12. Verfahren nach Anspruch 11, wobei das Betreiben eines jeden Fuzzyligik-Moduls mindestens einen der nachstehenden Schritte umfasst:

Klassifizieren von Glucose-Blut-Tendenzen in verschiedenen Kategorien;
Ermitteln der Wahrscheinlichkeit den Patienten in einem Ereignis der vielen Ereignisse als Funktion der Zeit anzutreffen; und
Anwenden eines Vorhersagemodells zum Vorhersagen der Glucosetendenz im Blut, basierend auf dem gemessenen Glucosewert.

**Revendications**

1. Système de surveillance automatique de traitement relatif au diabète d'un patient, le système (20) incluant une unité de commande (30) qui comprend :

   un premier module de processeur (34) pour analyser des données mesurées (106) indicatives de niveau de glucose sanguin (208) et générer des premières données traitées incluant des paramètres quantitatifs indicatifs au moins de tendances passées et futures dans le niveau de glucose sanguin ;
   un module de détection de repas (302) analysant les premières données traitées pour déterminer l'occurrence d'un événement de repas ou la probabilité que le patient soit dans un événement de repas en fonction du temps conformément à un modèle de niveaux de glucose ;
   un deuxième module de processeur (36), configuré pour recevoir des paramètres d'entrée correspondant aux données mesurées, lesdites premières données traitées, et des données de référence incluant une structure de réglages de règles, le deuxième module de processeur comprenant deux modules de logique floue (402, 502), dans lequel ledit deuxième module de processeur est configuré et utilisable pour alterner entre lesdits deux modules de logique floue,
   dans lequel l'un des deux modules de logique floue est associé à un événement de repas et l'autre des deux modules de logique floue est associé à un événement de repos, chacun des deux modules de logique floue étant configuré pour appliquer aux premières données traitées et aux données de référence un ensemble respectif de règles associées à l'événement respectif de manière à produire pour ledit événement au moins un paramètre de sortie pour indiquer au moins l'un parmi des paramètres de traitement d'insuline basale, d'insuline en bolus et de glucagon en bolus à modifier, dans lequel le module de logique floue associé à un événement de repas est utilisable si un événement de repas a été déterminé par le module de détection de repas, et sinon le module de logique floue associé à un événement de repos est utilisable, et
   un troisième module de processeur (38) étant configuré et utilisable pour recevoir ledit au moins un paramètre de sortie de l'un des deux modules de logique floue, des données individualisées relatives à l'historique de traitement du patient, une sensibilité à l'insuline / au glucagon à partir de données individualisées relatives au profil du patient et une quantité actuelle de glucagon et / ou d'insuline active dans le sang, et pour traiter ledit au moins un paramètre de sortie et lesdites données pour déterminer la quantité de dosage d'insuline et / ou de glucagon à administrer de manière à amener le niveau de glucose du patient à un niveau cible spécifique.

2. Système selon la revendication 1, dans lequel des paramètres d'entrée reçus par le deuxième module de processeur comprennent en outre au moins l'un parmi des paramètres de niveau actuel de glucose sanguin et des paramètres de niveau futur de glucose sanguin.

3. Système selon la revendication 1 ou 2, dans lequel ledit au moins un module de logique floue comprend un ensemble de règles associées à des facteurs de contribution désignant la probabilité qu'une condition d'un patient soit associée à une dite règle spécifique, et au moins un moteur flou utilisant une ou plusieurs fonctions élément modélisées pour traduire les paramètres d'entrée quantitatifs en ledit au moins un paramètre de sortie ; ledit deuxième module de processeur fournissant ainsi une commande pour varier une suggestion de traitement de sortie sur la base du au moins un paramètre de sortie du module de logique floue.

4. Système selon l'une quelconque des revendications précédentes, dans lequel ledit troisième processeur est configuré et utilisable pour déterminer ladite modification conformément à au moins l'une parmi une cible de glucose du profil du patient, la pharmacodynamie de l'insuline ou du glucagon du patient, et lesdites données mesurées.

5. Système selon l'une quelconque des revendications précédentes, **caractérisé par** au moins l'un parmi : (1) ladite unité de commande est associée à un dispositif d'injection de médicament est et configurée et utilisable pour commander le fonctionnement dudit dispositif d'injection de médicament ; et (2) l'unité de commande est utilisable pour mettre à jour et / ou calibrer lesdites données individualisées relatives au profil du patient pendant le traitement ou pendant une procédure de surveillance.

6. Système selon l'une quelconque des revendications précédentes, comprenant un émetteur-récepteur de données pour au moins l'un parmi :

   la réception d'au moins l'une desdites données de référence et desdites données mesurées ; et
   la transmission dudit au moins un paramètre de sortie de l'unité de commande à un dispositif d'injection de médicament.

EP 2 400 882 B1

**7.** Système selon l'une quelconque des revendications précédentes, dans lequel lesdites données individualisées relatives au profil du patient comprennent des paramètres choisis à partir d'au moins l'un parmi la sensibilité à l'insuline, la sensibilité au glucagon, le plan basal, des données associées à la pharmacocinétique de l'insuline / du glucagon, le niveau de cible ou le niveau de plage cible de glucose, et un modèle d'activité d'insuline / de glucagon.

**8.** Système selon l'une quelconque des revendications précédentes, dans lequel lesdites données individualisées relatives à l'historique de traitement du patient comprennent un régime d'administration d'insuline du patient délivré au patient à différentes heures de la journée.

**9.** Système selon la revendication 1, dans lequel les paramètres d'entrée reçus par le deuxième processeur comprennent en outre au moins l'un parmi : le temps écoulé entre des événements de repas détectés et le niveau de glucose sanguin par rapport audit événement de repas.

**10.** Système selon l'une quelconque des revendications précédentes, dans lequel lesdites données mesurées sont obtenues à un certain instant, lesdites données mesurées comprenant au moins l'un parmi des niveaux de glucose actuel et passé par rapport audit certain instant.

**11.** Procédé de surveillance automatique de traitement relatif au diabète d'un patient utilisant un système de commande qui est configuré pour exécuter les étapes de :

au niveau d'un premier module de processeur (34), analyser des données mesurées (106) indicatives de niveau de glucose sanguin (208) et générer des premières données traitées comprenant des paramètres quantitatifs indicatifs au moins de tendances passées et futures dans le niveau de glucose sanguin ;
au niveau d'un module de détection de repas (302), analyser les premières données traitées pour déterminer l'occurrence d'un événement de repas ou la probabilité que le patient soit dans un événement de repas en fonction du temps conformément à un modèle de niveaux de glucose ;
au niveau d'un deuxième module de processeur (36) comprenant deux modules de logique floue (402, 502), recevoir des paramètres d'entrée correspondant aux données mesurées, lesdites premières données traitées, et des données de référence incluant une structure de réglages de règles, et alterner entre lesdits deux modules logiques flous ;
au niveau des deux modules de logique floue, appliquer aux premières données traitées et aux données de référence un ensemble respectif de règles associées à l'événement respectif de manière à produire pour ledit événement au moins un paramètre de sortie pour indiquer au moins l'un parmi des paramètres de traitement d'insuline basale, d'insuline en bolus et de glucagon en bolus à modifier, dans lequel l'un des deux modules de logique floue est associé à un événement de repas et l'autre des deux modules de logique floue est associé à un événement de repos, et dans lequel le module de logique floue associé à un événement de repas est utilisable si un événement de repas a été déterminé par le module de détection de repas, et sinon le module de logique floue associé à un événement de repos est utilisable ; et
au niveau d'un troisième module de processeur (38), recevoir ledit au moins un paramètre de sortie de l'un des deux modules de logique floue, des données individualisées relatives à l'historique de traitement du patient, une sensibilité à l'insuline / au glucagon à partir de données individualisées relatives au profil du patient et une quantité actuelle de glucagon et / d'insuline active dans le sang, et traiter ledit au moins un paramètre de sortie et lesdites données pour déterminer la quantité de dosage d'insuline et / ou de glucagon à administrer de manière à amener le niveau de glucose du patient à un niveau cible spécifique.

**12.** Procédé selon la revendication 11, dans lequel l'utilisation de chaque module de logique floue comprend au moins l'un parmi :

classer des tendances de glucose sanguin en différentes catégories ;
déterminer la probabilité que le patient soit dans l'un parmi ladite pluralité d'événements en fonction du temps ; et
appliquer un modèle de prédiction pour prédire la tendance du glucose dans le sang sur la base du niveau de glucose mesuré.

Fig. 1

Fig. 2

**Control Unit** — 30

**34** Data Analysis

**115**

Calculate Past Trend - **110**

Predict BG values - **112**

Calculate Future Trend - **114**

Input parameters Created from 106,110 112 & 114 (Quantitative)

**118**

**Measured Data – 106**

To **38**

Current & history glucose levels

**Reference Data – 100**

Structured of rules settings - **105**

Patient Profile - **102**  → To **38**

Treatment History - **104**  → To **110, 114, 38**

**Control to Range Module** — 36

Fuzzy Logic Processing — **120**

Qualitative data of patient's condition — **122**

Multiple vectors of qualitative input parameters, each corresponding to a rule — **124**

Stacking and producing the deFuzzified Output Vector — **125**

To **38**

EP 2 400 882 B1

FIG. 2 (cont'd)

EP 2 400 882 B1

Fig. 3

─ Fuzzy Logic Parameters ───────────────────────────────────────

─ BG Values (Up and Normal) [mg/dL] ─────────

| | Low Bound | High Bound |
|---|---|---|
| Very Low | | |
| Low | | |
| Normal | | |
| Normal High | | |
| High | | |
| Very High | | |

─ Blood Glucose Trends [mg/dL*H] ─────────

| | Low Bound | High Bound |
|---|---|---|
| Steep Decent | | |
| Decent | | |
| Normal | | |
| Rise | | |
| Steep Rise | | |

─ Overlapping % BG Value ─────────

| Overlapping [%] | |
|---|---|

Fig. 4

Fig. 5

EP 2 400 882 B1

Fig. 6

Fig. 7A

Fig. 7B

Fig. 7C

Fig. 7D

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 2007149533 A

## Non-patent literature cited in the description

- **STEIL G ; PANTELEON A ; REBRIN K.** Closed-loop insulin delivery-the path to physiological glucose control. *Adv Drug Deliv Rev,* 2004, vol. 56, 125-144
- **PARKER R ; DOYLE FR ; PEPPAS N.** A model-based algorithm for blood glucose control in type I diabetic patients. *IEEE Trans Biomed Eng,* 1999, vol. 46, 148-157
- **HOVORKA R ; CHASSIN L ; WILINSKA M ; CANONICO V ; AKWI J ; FEDERICI M ; MASSI-BENEDETTI M ; HUTZLI I ; ZAUGG C ; KAUFMANN H.** Closing the loop: the adicol experience. *Diabetes Technol Ther,* 2004, vol. 6, 307-318
- **HOVORKA R ; CANONICO V ; CHASSIN L ; HAUETER U ; MASSI-BENEDETTI M ; ORSINI FEDERICI M ; PIEBER T ; SCHALLER H ; SCHAUPP L ; VERING T.** Nonlinear model predictive control of glucose concentration in subjects with type 1 diabetes. *Physiol Meas,* 2004, vol. 25, 905-920
- **MAGNI L ; RAIMONDO D ; BOSSI L ; DALLA MAN C ; DE NICOLAO G ; KOVATCHEV B ; COBELLI C.** Model Predictive Control of Type 1 Diabetes: An In Silico Trai. *J Diabetes Sci Technol,* 2007, vol. 1, 804-812
- Fuzzy Systems Engineering Towards Human-Centeric Computing. **PEDRYCZ W ; GOMIDE F.** Hoboken. John Wiley & Sons, Inc, 2007
- **SINCANANDAM SN ; SUMATHI S ; DEEPA SN.** Introduction to Fuzzy Logic using MATLAB. Verlag Berlin Heidelberg, Springer, 2007
- **SPARACINO G ; ZANDERIGO F ; CORAZZA S ; MARAN A ; FACCHINETTI A ; COBELLI C.** Glucose concentration can be predicted ahead in time from continuous glucose monitoring sensor time-series. *IEEE Trans Biomed Eng,* 2007, vol. 54, 931-937
- **MAGNI L ; RAIMONDO D ; DALLA MAN C ; BRETON M ; PATEK S ; DE NICOLAO G ; COBELLI C ; KOVATCHEV B.** Evaluating the Efficacy of Closed-Loop Glucose Regulation via Control-Variability Grid Analysis. *J Diabetes Sci Technol,* 2008, vol. 2, 630-635
- Standards of medical care in diabetes--2009. *Diabetes Care,* 2009, vol. 32 (1), 13-61